(19) Europäisches Patentamt
European Patent Office
Office européen des brevets

(11) **EP 1 475 074 A1**

(12) **DEMANDE DE BREVET EUROPEEN**

(43) Date de publication:
**10.11.2004 Bulletin 2004/46**

(51) Int Cl.$^7$: **A61K 7/09**, A61K 7/13, A61K 7/135

(21) Numéro de dépôt: **04101243.6**

(22) Date de dépôt: **25.03.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL LT LV MK**

(30) Priorité: **25.03.2003 FR 0350064**
**28.03.2003 FR 0350078**

(71) Demandeur: **L'OREAL**
**75008 Paris (FR)**

(72) Inventeurs:
- **LEGRAND, Frédéric**
  **92400, COURBEVOIE (FR)**
- **MILLEQUANT, Jean-Marie**
  **94100, SAINT MAUR DES FOSSES (FR)**

(74) Mandataire: **Poulin, Gérard**
**BREVALEX**
**3, rue du Docteur Lancereaux**
**75008 Paris (FR)**

(54) **Utilisation d'acides polycarboxyliques et de leurs sels comme agents complexants dans des compositions oxydantes pour la coloration, la décoloration ou la déformation permanente de fibres kératiniques**

(57) L'invention se rapporte à l'utilisation d'acides polycarboxyliques particuliers et de leurs sels en tant qu'agents complexants dans des compositions oxydantes destinées à la décoloration ou à la déformation permanente de fibres kératiniques, en particulier de fibres kératiniques humaines et plus spécialement de cheveux.

Elle se rapporte également à des compositions oxydantes pour la décoloration ou la déformation permanente de fibres kératiniques, qui renferment de tels agents complexants, ainsi qu'à des procédés et des dispositifs ou "kits" de décoloration ou de déformation permanente de fibres kératiniques.

EP 1 475 074 A1

## Description

### DOMAINE TECHNIQUE

La présente invention se rapporte à l'utilisation d'acides polycarboxyliques particuliers et de leurs sels en tant qu'agents complexants dans des compositions oxydantes destinées à la coloration, la décoloration ou à la déformation permanente de fibres kératiniques, en particulier de fibres kératiniques humaines et plus spécialement de cheveux.
Elle se rapporte également à des compositions oxydantes pour la coloration, la décoloration ou la déformation permanente de fibres kératiniques, qui renferment de tels agents complexants, ainsi qu'à des procédés et des dispositifs ou "kits" de coloration, de décoloration ou de déformation permanente de fibres kératiniques.

### ETAT DE LA TECHNIQUE ANTERIEURE

Pour colorer les fibres kératiniques, tels que les cheveux humains, il est connu d'utiliser des compositions tinctoriales contenant des précurseurs de colorant d'oxydation, en particulier des ortho- ou paraphénylènediamines, des ortho- ou paraaminophénols, des bases hétérocycliques, appelés généralement bases d'oxydation. Les précurseurs de colorants d'oxydation sont des précurseurs incolores ou faiblement colorés qui, associés à des produits oxydants, peuvent donner naissance par un processus de condensation oxydative à des composés colorés et colorants. On peut faire varier les nuances obtenues avec ces bases d'oxydation en les associant à des coupleurs ou modificateurs de coloration, ces derniers étant choisis parmi les métadiamines aromatiques, les métaaminophénols, les métadiphénols et certains composés hétérocycliques.
La coloration, engendrée par l'application sur les fibres à colorer de compositions tinctoriales en présence de produits oxydants, peut avoir pour fonction de colorer des cheveux gris mais également de modifier la couleur de cheveux naturels ou teints. Dans ce dernier cas, il est nécessaire, en particulier lorsque la couleur désirée est de plusieurs tons en dessous de la couleur initiale des cheveux à teindre, de procéder préalablement à la coloration, à une décoloration des cheveux à teindre.
Il existe aussi un besoin d'éclaircir donc de décolorer la chevelure sans pour autant vouloir la colorer à nouveau.
Pour décolorer des fibres kératiniques, on utilise généralement deux types de compositions : des compositions dites oxydantes car elles renferment un ou plusieurs agents aptes à oxyder la mélanine des cheveux et ainsi à la solubiliser pour en obtenir l'élimination totale ou partielle, et des compositions dites au contraire réductrices car elles contiennent un ou plusieurs agents réducteurs du type acide ascorbique ou thiols, ces dernières étant plus spécialement destinées à la décoloration de cheveux ayant été antérieurement teints avec des pigments exogènes.
En ce qui concerne la déformation permanente, il est usuel d'appliquer sur la chevelure préalablement mise sous tension, par exemple à l'aide de bigoudis si la déformation recherchée est une frisure, une composition contenant un ou plusieurs agents réducteurs de manière à induire l'ouverture des ponts disulfures formés par les résidus cystéine de la kératine des cheveux, puis, généralement après un rinçage, de réoxyder la chevelure pour en fixer la déformation, par le biais de l'application d'une composition oxydante.
Concernant les compositions oxydantes nécessaires à la mise en oeuvre de l'étape de fixation, on fait le plus souvent appel, dans la pratique, à des compositions à base d'eau oxygénée, de bromate de sodium ou de persels comme le perborate de sodium.
Qu'elles soient destinées à la coloration, la décoloration ou à la déformation permanente, les compositions oxydantes contiennent en principe un agent complexant destiné à complexer les cations métalliques susceptibles de se trouver à l'état de traces dans ces compositions, ainsi que ceux pouvant être présents sur les cheveux et provenant de l'air ambiant, de l'eau avec laquelle ces derniers ont été lavés ou encore des shampoings ou autres produits capillaires avec lesquels ils ont été traités.
Il est, en effet, très important de neutraliser ces cations métalliques, dans la mesure où ils sont susceptibles de catalyser les réactions d'oxydation des fibres capillaires et ce, de façon non contrôlée, ce qui peut se traduire par des effets indésirables sévères tels qu'une cassure des cheveux ou une brûlure du cuir chevelu.
Actuellement, les agents complexants les plus couramment utilisés dans les compositions oxydantes pour la coloration, la décoloration ou la déformation permanente de fibres kératiniques sont l'acide éthylènediamine tétraacétique (EDTA) et ses dérivés comme l'acide diéthylènetriamine pentaacétique (DPTA), généralement dans des proportions pondérales de l'ordre de 0,1 à 1%.
Toutefois, dans le cadre de ses travaux, la Demanderesse a constaté que l'EDTA et ses dérivés présentent, dans ce type de compositions, des propriétés insuffisantes. Ces constatations, qui sont corroborées par les résultats obtenus par d'autres équipes de recherche, justifient de trouver de nouveaux agents complexants.
Un agent complexant destiné à entrer dans la constitution de compositions oxydantes pour la coloration, la décoloration ou la déformation permanente de fibres kératiniques, doit satisfaire à de nombreuses exigences. En effet,

outre qu'il doit présenter un pouvoir complexant élevé vis-à-vis des métaux de manière à supprimer ou, à tout le moins, réduire le plus possible le risque d'une catalyse des réactions d'oxydation des fibres kératiniques par les traces métalliques susceptibles d'être présentes dans ces compositions et sur ces fibres, il doit être compatible, et notamment ne pas réagir, avec les autres constituants, et en particulier avec le ou les agents oxydants. Il doit également être stable en solution, les compositions oxydantes étant, en effet, appliquées généralement sur les fibres kératiniques sous forme de solutions. Il doit aussi être d'une innocuité totale pour ces fibres et pour la peau, et notamment être dénué de tout caractère allergène.

Il est, en outre, souhaitable, dans un souci de respect de l'environnement, qu'il soit biodégradable, et que son coût de production ou d'achat autorise son utilisation dans des compositions destinées à être vendues, non seulement à des professionnels, mais également dans la distribution grand public.

Or, après de longues recherches menées sur la question, la Demanderesse a découvert que, de manière surprenante, certains polyacides et leurs sels sont susceptibles de répondre à toutes ces exigences et de représenter, par conséquent, des agents complexants de choix dans des compositions oxydantes pour la coloration, la décoloration et la déformation permanente de fibres kératiniques.

Et c'est cette découverte qui est à la base de l'invention.

## EXPOSÉ DE L'INVENTION

Ainsi, l'invention a, en premier lieu pour objet l'utilisation, en tant qu'agent complexant de cations métalliques présents dans une composition oxydante, comprenant au moins un oxydant, pour la décoloration, la coloration ou la déformation permanente de fibres kératiniques, d'au moins un composé répondant à la formule (I) suivante :

$$R\text{-}N\text{-}(CH(R')CO_2X)_2 \qquad (I)$$

dans laquelle :

- R représente un atome d'hydrogène ou un groupe $-CH(CO_2X)-(CH_2)_2CO_2X$, $-CH(CH_3)-CO_2X$ ou $-(CH_2)_2-N(COR'')-CH_2-CO_2X$ ;
- R' représente un groupe $-CH_2CO_2X$ lorsque R représente un atome d'hydrogène, tandis que R' représente un atome d'hydrogène lorsque R est différent d'un atome d'hydrogène ; et
- R'' représente un groupe alkyle, linéaire ou ramifié, comportant de 1 à 30 atomes de carbone, ou bien un groupe alkyle cyclique comportant de 3 à 30 atomes de carbone ;
- X représente un atome d'hydrogène ou un cation monovalent ou divalent issu d'un métal alcalin, d'un métal alcalino-terreux, d'un métal de transition, d'une amine organique ou un cation ammonium.

Ainsi, les agents complexants utilisés dans le cadre de l'invention, correspondent à des composés acides polycarboxyliques et aux carboxylates correspondants.

Plus précisément, les agents complexants correspondent à :

- des composés comprenant quatre fonctions acides carboxyliques ou carboxylates, lorsque R représente un atome d'hydrogène et R' représente un groupe $-CH_2-CO_2X$, ou lorsque R représente le groupe $-CH(CO_2X)-(CH_2)_2-CO_2X$ et R' représente un atome d'hydrogène ;
- des composés comprenant trois fonctions acides carboxyliques ou carboxylates, lorsque R représente le groupe $-CH(CH_3)-CO_2X$ et R' représente un atome d'hydrogène, ou lorsque R représente un groupe $-(CH_2)_2-N(COR'')-CH_2-CO_2X$ et R' représente un atome d'hydrogène.

Conformément à l'invention, lorsque le ou les composés de formule (I) sont des carboxylates, alors le cation monovalent ou divalent est, de préférence, choisi dans le groupe constitué par les cations monovalents de métaux alcalins, les cations divalents de métaux alcalino-terreux, les cations divalents de métaux de transition et les cations monovalents issus d'amines organiques ou les cations ammonium.

A titre d'exemples de cations de métaux alcalins, on peut notamment citer le sodium ($Na^+$) et le potassium ($K^+$), tandis qu'à titre d'exemples de cations de métaux alcalino-terreux, on peut notamment citer le calcium ($Ca^{2+}$) et le magnésium ($Mg^{2+}$) .

Au sens de la présente invention, on entend par "métal de transition", un métal comportant une sous-couche d incomplète, plus particulièrement à l'état d'oxydation II, tel que le cobalt ($Co^{2+}$), le fer ($Fe^{2+}$) , le manganèse ($Mn^{2+}$) , le zinc ($Zn^{2+}$) et le cuivre ($Cu^{2+}$).

En ce qui concerne les cations monovalents d'amines organiques, on peut citer les cations d'amine primaire, secondaire ou tertiaire, ou encore d'alcanolamine.

Lesdites amines présentent un ou plusieurs radicaux, identiques ou non, de type alkyle, linéaire ou ramifié en $C_1$ à $C_{20}$, comprenant éventuellement un hétéroatome comme l'oxygène.

Pour ce qui a trait aux cations monovalents d'ammonium quaternaires, ces derniers comprennent trois radicaux, identiques ou non, choisis parmi l'hydrogène, un radical alkyle, linéaire ou ramifié en $C_1$ à $C_{20}$, comprenant éventuellement un hétéroatome comme l'oxygène.

Conformément à l'invention, lorsque le ou les composés de formule (I) sont des carboxylates, alors le cation monovalent ou divalent est, de préférence, choisi dans le groupe constitué par les cations de métaux alcalins, les cations de métaux alcalino-terreux et les cations divalents de métaux de transition, les cations issus d'amines organiques ou d'ammonium.

Le ou les composés de formule (I) sont, de préférence, choisis dans le groupe constitué par l'acide méthylglycine diacétique, l'acide N-lauroyl-N,N',N'-tri-acétique éthylènediamine, l'acide iminodisuccinique, l'acide N,N-dicarboxyméthyl L-glutamique, leurs sels de métaux alcalins, leurs sels de métaux alcalino-terreux, leurs sels de métaux de transition, leurs sels d'amines organiques, leurs sels d'ammonium, et leurs mélanges.

L'acide méthylglycine diacétique, l'acide N-lauroyl-N,N',N'-triacétique éthylènediamine, l'acide iminodisuccinique, l'acide N,N-dicarboxyméthyl L-glutamique et leurs sels sont respectivement représentés par les formules (II), (III), (IV), (V) suivantes :

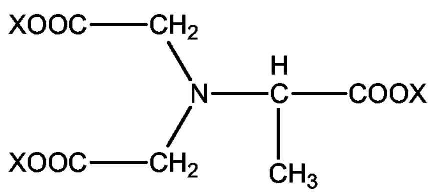

(II)

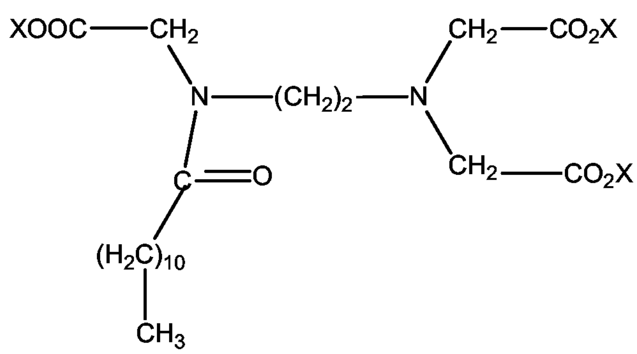

(III)

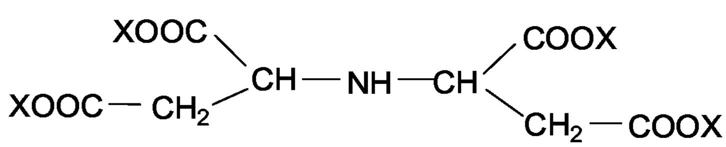

(IV)

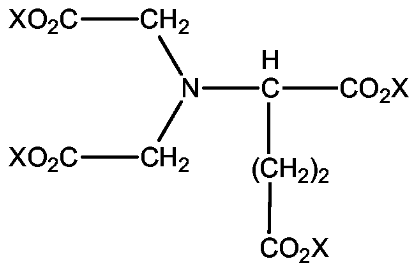

(V)

dans lesquelles X est tel que défini précédemment, X correspondant, de préférence, à H ou Na.

Ces composés sont notamment disponibles auprès des sociétés BASF, DOW CHEMICAL, HAMPSHIRE, BAYER et SHOWA DENKO.

Plus particulièrement, on préfère l'acide méthylglycine-diacétique, et ses sels de sodium.

De préférence, le ou les composés de formule (I) représentent de 0,001 à 10% en poids et, mieux encore, de 0,001 à 5% en poids du poids total de la composition oxydante. Les pourcentages en poids sont exprimés par rapport à la forme acide du ou des composés de formule (I).

Conformément à l'invention, la composition oxydante comprend un ou plusieurs agents oxydants, qui peuvent être choisis parmi les agents oxydants classiquement utilisés dans les compositions cosmétiques destinées à la coloration, la décoloration et la déformation permanente des fibres kératiniques. Parmi les oxydants classiquement utilisés, on peut citer le peroxyde d'hydrogène, le peroxyde d'urée, les bromates de métaux alcalins, les persels tels que les perborates, percarbonates et persulfates, les peracides.

Selon l'invention, il est particulièrement avantageux d'utiliser comme agents complexants l'acide méthylglycinediacétique, l'acide iminodisuccinique, éventuellement sous forme de sels en association avec l'eau oxygénée.

Selon l'invention, la composition oxydante peut comprendre en plus du ou des composés de formule (I) et du ou des agents oxydants, un ou plusieurs composés choisis parmi :

(A) les polymères conditionneurs cationiques ou amphotères ;
(B) les polymères amphiphiles non ioniques, anioniques, cationiques ou amphotères, comportant au moins une chaîne hydrophobe ;
(C) les agents tensioactifs anioniques, non-ioniques, cationiques, amphotères ou zwittérioniques ;
(D) les agents d'ajustement de la rhéologie autres que les polymères (B) ;
(E) les agents d'ajustement du pH (5) ;
(F) les solvants (7).

(A) <u>les polymères conditionneurs cationiques ou amphotères</u> :

Au sens de la présente invention, on entend par "polymère conditionneur cationique", tout polymère qui comprend des groupes cationiques ou des groupes ionisables en groupes cationiques et qui permet d'améliorer les propriétés cosmétiques des fibres kératiniques, en particulier le démêlage, la douceur, la brillance, le volume.

Les polymères conditionneurs cationiques ou amphotères convenables sont de manière avantageuse, choisis parmi ceux déjà connus en soi comme améliorant les propriétés cosmétiques des cheveux, à savoir notamment ceux décrits dans les brevets et demandes de brevet EP 337 354, FR 2 270 846, FR 2 383 660, FR 2 598 611, FR 2 470 596, FR 2 519 863, FR 2 788 974, FR 2 788 976 pour avoir une liste de ces composés.

Cependant, à titre d'exemples plus précis de polymères conditionneurs cationiques, on peut notamment citer les polymères cationiques comprenant au moins des groupements amine primaire, secondaire, tertiaire et/ou quaternaire pouvant, soit faire partie de la chaîne principale polymère, soit être portés par un substituant latéral directement relié à celle-ci.

Ainsi, on peut citer :

(1) les copolymères d'acrylamide et de diméthyl-amino-éthyl méthacrylate quaternisé au sulfate de diméthyle ou avec un halogénure de diméthyle (Hercofloc de Hercules) ; les copolymères d'acrylamide et de chlorure de méthacryloyloxy-éthyl-triméthylammonium (Bina Quat P 100 de Ciba Geigy) ; le copolymère d'acrylamide et de méthosulfate de méthacryloyloxy-éthyl-triméthylammonium (Reten de Hercules) ; les copolymères vinylpyrrolidone / acrylate ou méthacrylate de dialkylaminoalkyle quaternisés ou non (gamme Gafquat d'ISP ; Copolymer 845, 958 et 937 d'ISP) ; les terpolymères méthacrylate de diméthyl amino éthyle/ vinylcaprolactame/ vinylpyrrolidone (Gaffix VC 713 d'ISP) ; les copolymères vinylpyrrolidone / méthacrylamidopropyl dimethylamine (Styleze CC 10 d'ISP) ;

les copolymères vinylpyrrolidone / méthacrylamide de diméthyl-aminopropyle quaternisés (Gafquat HS 100 d'ISP) ;

(2) les dérivés d'éthers de cellulose comportant des groupements ammonium quaternaire tels que décrits dans FR 1 492 597. Ces polymères sont également définis dans le dictionnaire CTFA comme des ammoniums quaternaires d'hydroxyéthylcellulose ayant réagi avec un époxyde substitué par un groupement triméthylammonium ;

(3) les dérivés de cellulose cationiques tels que les copolymères de cellulose ou les dérivés de cellulose greffés avec un monomère hydrosoluble d'ammonium quaternaire, et décrits notamment dans US 4,131,576, tels que les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamido propyl-triméthylammonium, de diméthyldiallylammonium ;

(4) les polysaccharides cationiques décrits plus particulièrement dans les brevets US 3,589,578, US 4,031,307, tels que les gommes de guar contenant des groupements cationiques trialkylammonium. On utilise par exemple des gommes de guar modifiées par un sel, comme le chlorure notamment, de 2,3-époxypropyl triméthylammonium ;

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR 2 162 025, FR 2 280 361 ;

(6) les polyaminoamides solubles dans l'eau préparés en particulier par polycondensation d'un composé acide avec une polyamine, éventuellement réticulés, éventuellement alcoylés ou s'ils comportent une ou plusieurs fonctions amines tertiaires, quaternisées. Ces polymères sont notamment décrits dans FR 2 252 840 et FR 2 368 508 ;

(7) les dérivés de polyaminoamides résultant de la condensation de polyalkylènes polyamines avec des acides polycarboxyliques suivie d'une alcoylation par des agents bifonctionnels. On peut citer par exemple les polymères acide adipiquedialkylamino hydroxyalkyl dialkylène triamine dans lesquels le radical alkyle est en $C_1$-$C_4$. De tels polymères sont notamment décrits dans FR 1 583 363 ;

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en $C_3$-$C_8$, puis avec l'épichlorhydrine. De tels polymères sont notamment décrits dans US 3,227,615, US 2,961,347 ;

(9) les cyclopolymères d'alkyl diallyl amine ou de dialkyl diallyl ammonium, sous forme d'homopolymères ou de copolymères, tels que décrits dans FR 2 080 759 et dans son certificat d'addition n°2 190 406 ;

(10) les polymères de diammonium quaternaire tels que décrits dans FR 2 320 330, FR 2 270 846, FR 2 316 271, FR 2 336 434, FR 2 413 907, US 2,273,780, US 2,375,853, US 2,388,614, US 2,454,547, US 3,206,462, US 2,261,002, US 2,271,378, US 3,874,870, US 4,001,432, US 3,929,990, US 3,966,904, US 4,005,193, US 4,025,617, US 4,025,627, US 4,025,653, US 4,026,945, US 4,027,020 ; par exemple, on peut citer ceux comprenant les motifs récurrents suivants:

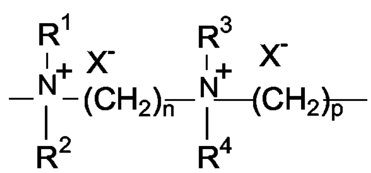

dans laquelle les radicaux $R^1$, $R^2$, $R^3$ et $R^4$, identiques ou différents, désignent un radical alkyle ou hydroxyalkyle en $C_1$-$C_4$, n et p sont des nombres entiers variant de 2 à 20 et, $X^-$ est un anion dérivé d'un acide minéral ou organique ;

(11) les polymères de poly(ammonium quaternaire) constitués de motifs récurrents de formule :

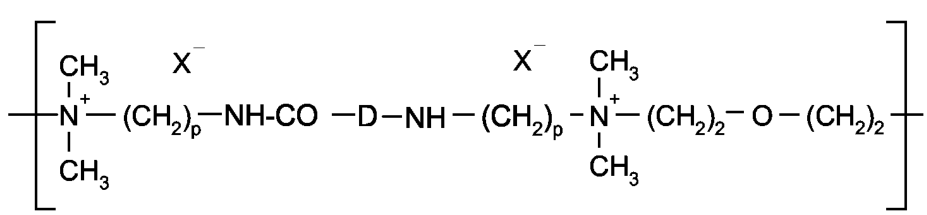

dans laquelle p désigne un nombre entier variant de 1 à 6 environ, D peut être nul ou peut représenter un groupement $-(CH_2)_r$ -CO- dans lequel r désigne un nombre égal à 4 ou à 7, $X^-$ est un anion. De tels polymères peuvent être préparés selon les procédés décrits dans US 4,157,388, US 4,702,906, US 4,719,282, EP 122 324 ;
(12) les polymères quaternaires de vinylpyrrolidone et de vinylimidazole ;
(13) les polyamines du type polyethyleneglycol (15) Tallow Polyamine(dénomination du dictionnaire CTFA) ;
(14) les polymères réticulés de sels de méthacryloyloxyalkyl ($C_1$-$C_4$) trialkyl ($C_1$-$C_4$) ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/ chlorure de méthacryloyloxyéthyl triméthylammonium (20/80 en poids) sous forme de dispersion contenant 50 % en poids dudit copolymère dans de l'huile minérale (Salcare® SC 92 de Ciba). On peut également utiliser un homopolymère réticulé du chlorure de méthacryloyl oxyéthyl triméthylammonium contenant environ 50 % en poids de l'homopolymère dans de l'huile minérale ou dans un ester liquide (Salcare® SC 95, SC 96 de Ciba).

D'autres polymères conditionneurs cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Le ou les polymères conditionneurs amphotères susceptibles d'être présents dans la composition oxydante peuvent, eux, notamment être choisis parmi ceux comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques ; ou bien K et M peuvent désigner des groupements dérivant de monomères zwittérioniques de carboxybétaïnes ou de sulfobétaïnes ; ou encore K et M désignent une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ; ou bien K et M font partie d'une chaîne d'un polymère à motif éthylène $\alpha,\beta$-dicarboxylique dont l'un des groupements carboxyliques a réagi avec une polyamine comportant un ou plusieurs groupements amine primaire ou secondaire.

Les polymères conditionneurs amphotères répondant à la définition donnée ci-dessus plus particulièrement préférés, sont choisis parmi les polymères suivants :

(1) les polymères résultant de la copolymérisation d'un monomère dérivé d'un composé vinylique portant un groupement carboxylique tel que plus particulièrement l'acide (méth)acrylique, l'acide maléique, l'acide alpha-chloracrylique, ou encore un sel de dialkyldiallylammonium tel que le chlorure de diméthyldiallylammonium, et d'un monomère basique dérivé d'un composé vinylique substitué contenant au moins un atome basique tel que plus particulièrement les dialkyl-amino-alkyl-méthacrylate et acrylate, les dialkyl-amino-alkyl-méthacrylamide et acrylamide, comme décrits dans US 3,836,537. On peut citer le copolymère acrylate de sodium / chlorure d'acrylamido propyl trimethyl ammonium (Polyquart KE 3033 de Cognis), le copolymère acide acrylique/ chlorure de diméthyldiallyl ammonium (Merquat 280, 295, Plus 3330, de Nalco) ;
(2) les polymères comportant des motifs dérivant a) d'au moins un monomère choisi parmi les (méth)acrylamides N-substitués par un radical alkyle, notamment en $C_2$-$C_{12}$, b) d'au moins un monomère acide contenant un ou plusieurs groupements carboxyliques réactifs (par exemple acides (méth)acrylique, crotonique, itaconique, et les monoesters des acides ou anhydrides maléique, fumarique), et c) d'au moins un monomère basique tel que des esters à substituant amine primaire, secondaire, tertiaire et quaternaire des acides (méth)acrylique, fumarique, maléique, et le produit de quaternisation du méthacrylate de diméthylamino-éthyle avec le sulfate de diméthyle ou diéthyle. On utilise particulièrement les copolymères octylacrylamide / acrylate / butylaminoéthyl méthacrylate (Amphomer ou Lovocryl 47 par la société National Starch) ;
(3) les polyaminoamides réticulés et partiellement ou totalement alcoylés, dérivant de polyaminoamides de formule générale $-[CO-R^5-CO-Z]-$ dans laquelle $R^5$ est un radical divalent dérivé d'un acide dicarboxylique saturé ou non (par exemple les acides adipique, triméthyl-2,2,4-adipique et triméthyl-2,4,4-adipique, téréphtalique, itaconique), d'un acide monocarboxylique insaturé (comme l'acide (méth)acrylique), d'un ester d'alcool en $C_1$-$C_6$ des acides précités ou d'un radical dérivant de l'addition de l'un de ces acides avec une amine bis-primaire ou - secondaire, et Z désigne un radical d'une polyalkylènepolyamine bis-primaire, mono ou bis-secondaire. De préférence, Z représente entre 60 et 100 moles %, le radical $-NH-[(CH_2)_x-NH]_p-$ avec x=2 et p=2 ou 3, ou x=3 et p=2 ; ce radical dérivant de la diéthylène triamine, de la triéthylène tétraamine ou de la dipropylène triamine; entre 0 et 40 moles % le radical ci-dessus, dans lequel x=2 et p=1 et qui dérive de l'éthylènediamine, ou le radical dérivant de la pipérazine $-N[CH_2CH_2]_2N-$ ; entre 0 et 20 moles %, le radical $-NH-(CH_2)_6-NH-$ dérivant de l'hexaméthylène diamine. L'agent réticulant de ces polymères est un agent bifonctionnel choisi parmi les épihalohydrines, les diépoxydes,

les dianhydrides, les dérivés bis insaturés, et alcoylés par action d'acide acrylique, d'acide chloracétique ou d'une alcane sultone ou de leurs sels de métaux alcalins ;

(4) les polymères comportant au moins des motifs zwittérioniques, comme par exemple le copolymère de méthacrylate de butyle / méthacrylate de diméthyl carboxy-méthyl-ammonio-éthyle (Diaformer Z301, Sandoz) ;

(5) les polymères dérivés du chitosane comportant des motifs monomères répondant aux formules (I), (II), (III) suivantes :

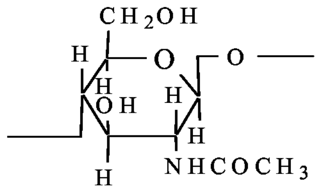
(I)

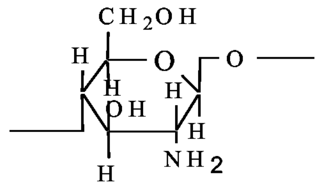
(II)

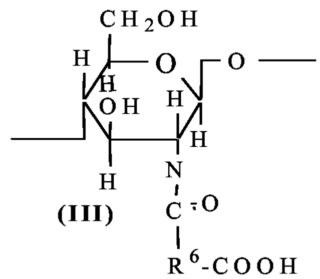
(III)

avec (I) représentant de 0 à 30%, (II) de 5 à 50% et (III) de 30 à 90% dans lequel $R^6$ représente un radical de formule :

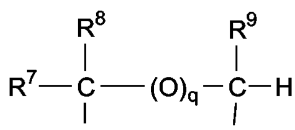

dans laquelle q désigne zéro ou 1 ; et si q=0, les $R^7$, $R^8$, $R^9$, identiques ou différents, représentent un hydrogène, un groupement méthyle, hydroxyle, acétoxy, amino, mono- ou di-alkylamine éventuellement interrompus par un ou plusieurs atomes d'azote et/ou éventuellement substitués par un ou plusieurs groupes amine, hydroxyle, carboxyle, alkylthio éventuellement porteur d'un groupe amino, sulfonique ; ou si q=1, les $R^7$, $R^8$, $R^9$, identiques ou différents, représentent un hydrogène, ainsi que les sels formés par ces composés avec des bases ou des acides,

(6) les polymères dérivés de la N-carboxyalkylation du chitosane comme le N-carboxyméthyl chitosane ou le N-carboxybutyl chitosane (Evalsan de Jan Dekker) ;

(7) les polymères tels que décrits dans FR 1 400 366 :

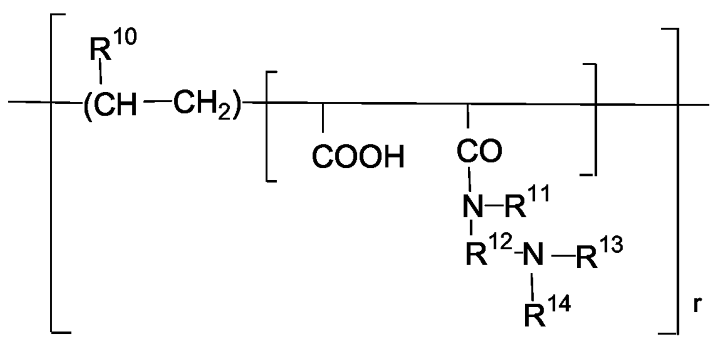

dans laquelle $R^{10}$ est un hydrogène, $CH_3O$-, $CH_3CH_2O$-, phényle, $R^{11}$ et $R^{14}$, identiques ou différents, représentent un hydrogène, un radical alkyle (méthyle, éthyle), $R^{13}$ représente un radical alkyle (méthyle, éthyle) ou un radical de formule $-R^{12}-N(R^{14})_2$, $R^{12}$ représentant

$-(CH_2)_2-$, $-(CH_2)_3-$, $-CH_2-CH(CH_3)-$, ainsi que les homologues supérieurs de ces radicaux et contenant jusqu'à 6 atomes de carbone, r est tel que le poids moléculaire est compris entre 500 et 6000000 et de préférence entre 1000 et 1000000 ;

(8) les polymères amphotères du type - $D^1$-X-$D^1$-X- choisis parmi:

a) les polymères obtenus par action de l'acide chloracétique ou le chloracétate de sodium sur les composés comportant au moins un motif de formule $-D^1-X-D^1-X-D^1-$ où $D^1$ désigne un radical pipérazinyle et X désigne le symbole E ou E', E ou E' identiques ou différents désignent un radical bivalent qui est un radical alkylène à chaîne droite ou ramifiée comportant jusqu'à 7 atomes de carbone dans la chaîne principale substituée ou non par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
b) les polymères de formule $-D^1-X-D^1-X-$ où $D^1$ désigne un radical pipérazinyle et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude ;

(9) les copolymères alkyl ($C_1$-$C_5$) - vinyléther/anhydride maléique modifié partiellement par semi-amidification avec une N,N-dialkylaminoalkylamine telle que la N,N-diméthyl-amino-propylamine ou par semi-estérification avec une N,N-dialcanolamine. Ces copolymères peuvent également comporter d'autres comonomères vinyliques tels que le vinylcaprolactame.

Parmi les polymères conditionneurs cationiques ou amphotères utilisables, on préfère notamment :

(i) parmi les cationiques :

- l'homopolymère de chlorure de diméthyldiallylammonium (Merquat 100 de Nalco);
- les copolymères de chlorure de diméthyldiallylammonium et d'acrylamide (Merquat 2200 de Nalco);
- les polymères de type poly(ammonium quaternaire) préparés et décrits dans FR 2 270 846, constitués de motifs récurrents de formules (W) et (U) suivantes :

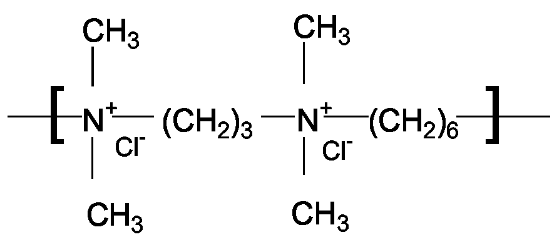

**(W)**

et notamment ceux dont le poids moléculaire, déterminé par chromatographie par perméation de gel, est compris entre 9500 et 9900;

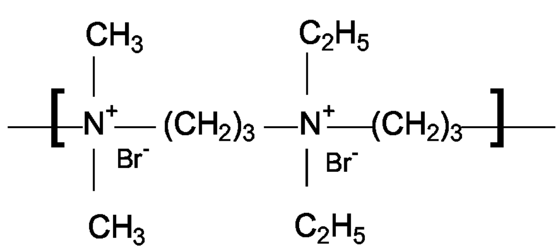

**(U)**

et notamment ceux dont le poids moléculaire, déterminé par chromatographie par perméation de gel, est d'environ 1200;
- les polymères de type poly(ammonium quaternaire) de la famille (11) avec $X^-$ désignant le chlore, et notamment ceux dont la masse moléculaire moyenne en poids est inférieure à 100 000, de préférence inférieure ou égale

à 50 000;

(ii) parmi les polymères amphotères :

- le copolymère chlorure de diméthyldiallylammonium / acide acrylique (80/20) (Merquat 280 de Nalco- dénomination CTFA : Polyquaternium 22) ;
- le copolymère chlorure de diméthyldiallylammonium /acide acrylique (95/5) (Merquat 295 de Nalco) ;
- le copolymère de chlorure de méthacrylamidopropyl trimonium, d'acide acrylique et d'acrylate d'éthyle (Merquat 2001 de Nalco - dénomination CTFA : Polyquaternium 47) ;
- le terpolymère acrylamide/chlorure de diméthyldiallyl ammonium/acide acrylique (Merquat Plus 3330 de Nalco -dénomination CTFA : Polyquaternium 39).

Lorsque la composition oxydante comprend un u plusieurs polymères conditionneurs cationiques ou amphotères, alors celui-ci ou ceux-ci représentent généralement de 0,01 à 10% en poids et, mieux encore, de 0,05 à 5% du poids total de cette composition.

(B) les polymères amphiphiles non ioniques, anioniques, cationiques ou amphotères, comportant une chaîne hydrophobe :

Plus particulièrement, la chaîne hydrophobe est une chaîne hydrocarbonée, saturée ou non, aromatique ou non, linéaire ou ramifiée, en $C_6$-$C_{30}$, comprenant éventuellement un ou plusieurs motifs oxyalkylénés (oxyéthylénés et/ou oxypropylénés).
Parmi les polymères amphiphiles cationiques comportant une chaîne hydrophobe, on peut trouver des polyuréthannes cationiques ou des copolymères cationiques comprenant des motifs vinyllactame et en particulier vinylpyrrolidone.
De préférence, les polymères amphiphiles comportant une chaîne hydrophobe sont de nature non ionique ou anionique.
A titre d'exemples de polymères amphiphiles non ioniques à chaîne hydrophobe, on peut citer entre autres :

(1) les celluloses modifiées par des groupements comportant au moins une chaîne hydrocarbonée, saturée ou non, linéaire ou ramifiée, en $C_6$-$C_{30}$, comme les hydroxyéthylcelluloses modifiées par des groupements comportant au moins une chaîne hydrophobe telle que définie auparavant, comme notamment Natrosol Plus Grade 330 CS (alkyles en $C_{16}$ - commercialisé par la société Aqualon) ; Bermocoll EHM 100 (commercialisé par la société Berol Nobel), Amercell Polymer HM-1500 (hydroxyéthylcellulose modifiée par un groupement polyéthylène glycol (15) éther de nonylphénol - commercialisé par la société Amerchol) ;
(2) les hydroxypropylguars modifiés par des groupements comportant au moins une chaîne hydrophobe telle que définie, par exemple Jaguar XC-95/3 (chaîne alkyle en $C_{14}$ - commercialisé par la société Rhodia Chimie) ; Esaflor HM 22 (chaîne alkyle en $C_{22}$ - commercialisé par la société Lamberti) ; RE210-18 (chaîne alkyle en $C_{14}$) et RE205-1 (chaîne alkyle en $C_{20}$) commercialisés par la société Rhodia Chimie ;
(3) les copolymères de vinylpyrrolidone et de monomères hydrophobes à chaîne hydrophobe telle que définie auparavant comme par exemple Antaron ou Ganex V216 (copolymères vinylpyrrolidone / hexadécène) ; Antaron ou Ganex V220 (copolymères vinylpyrrolidone / eicosène), commercialisés par la société I.S.P ;
(4) les copolymères de (méth)acrylates d'alkyles en $C_1$-$C_6$ et de monomères amphiphiles comportant une chaîne hydrophobe ;
(5) les copolymères de (méth) acrylates hydrophiles et de monomères hydrophobes comportant au moins une chaîne hydrophobe, tels que par exemple le copolymère méthacrylate de polyéthylèneglycol / méthacrylate de lauryle ;
(6) les polymères à squelette aminoplaste éther possédant au moins une chaîne grasse, tels que les composés Pure Thix commercialisés par la société Süd-Chemie ;
(7) les polyéthers polyuréthannes, linéaires (structure à blocs), greffés ou en étoile, comportant dans leur chaîne, au moins une séquence hydrophile, généralement polyoxyéthylénée et pouvant comprendre entre 50 et 1000 motifs oxyéthylène environ, et au moins une séquence hydrophobe, qui peut comprendre des groupements aliphatiques seuls, éventuellement combinés à des enchaînements cycloaliphatiques et/ou aromatiques. De préférence, les polyéthers polyuréthannes comportent au moins deux chaînes hydrophobes hydrocarbonées en $C_6$-$C_{30}$, séparées par une séquence hydrophile ; les chaînes hydrophobes pouvant être des chaînes pendantes ou des chaînes à l'une ou plusieurs des extrémités de la ou des séquences hydrophiles.

Les polyéthers polyuréthannes comportent une liaison uréthanne entre les séquences hydrophiles, mais peuvent aussi comprendre des séquences hydrophiles liées aux séquences lipophiles par d'autres liaisons chimiques.

Les polyéthers polyuréthannes sont en particulier ceux décrits dans l'article de G. Fonnum, J. Bakke et Fk. Hansen - Colloid Polym. Sci.271, 380-389 (1993). A titre d'exemples de polyéthers polyuréthannes, on peut citer Nuvis FX 1100 (désignation I.N.C.I. européenne et américaine "Steareth-100/PEG-136/H.M.D.I. Copolymer" commercialisé par la société Servo Delden) ; Rheolate 205, 208 , 204 ou 212 (commercialisés par la société Rheox) ; Elfacos T210 (chaîne alkyle en $C_{12}$- $C_{14}$) Elfacos T212 (chaîne alkyle en $C_{18}$) commercialisés par la société Akzo.

Les polymères amphiphiles anioniques à chaîne hydrophobe, susceptibles d'être mis en oeuvre comportent au moins à titre de chaîne hydrophobe, une chaîne hydrocarbonée, saturée ou non, aromatique ou non, linéaire ou ramifiée, en $C_8$-$C_{30}$.

Plus particulièrement les polymères amphiphiles anioniques comportant au moins une chaîne hydrophobe, réticulés ou non, comprennent au moins un motif hydrophile dérivé d'un ou de plusieurs monomères à insaturation éthylénique portant une fonction acide carboxylique, ou une fonction acide sulfonique, libre ou partiellement ou totalement neutralisée, et au moins un motif hydrophobe dérivé d'un ou de plusieurs monomères à insaturation éthylénique portant une chaîne latérale hydrophobe, et éventuellement au moins un motif de réticulation dérivés d'un ou plusieurs monomères polyinsaturés.

Des polymères amphiphiles anioniques du type décrit ci-dessus sont décrits et préparés, par exemple dans les brevets US 3,915,921 et US 4,509,949 (copolymères d'acide (méth)acrylique et de (méth)acrylates d'alkyles en $C_{10}$-$C_{30}$) ou dans le brevet EP 216 479 (copolymères d'acide (méth)acrylique et d'éthers allyliques d'alcools gras).

Les polymères amphiphiles comportant au moins un groupement sulfonique, sous forme libre ou partiellement ou totalement neutralisée et au moins une partie hydrophobe sont par exemple décrits dans FR 00 16954 et FR 01 00328 dont le contenu fait partie intégrante de la présente invention.

On peut citer plus particulièrement parmi eux le copolymère acide acrylamido-2-méthyl-2-propanesulfonique (AMPS)/n-dodécylacrylamide neutralisé par la soude, le copolymère réticulé par du méthylène-bis-acrylamide constitué de 75% en poids de motifs AMPS neutralisés par $NH_3$ et de 25% en poids de motifs acrylate de Genapol T-250, le copolymère réticulé par du méthacrylate d'allyle constitué de 90% en poids de motifs AMPS neutralisés par $NH_3$ et de 10% en poids de motifs méthacrylate de Genapol T-250, ou le copolymère réticulé par du méthacrylate d'allyle constitué de 80% en poids de motifs AMPS neutralisés par $NH_3$ et de 20% en poids de motifs méthacrylate de Genapol T-250.

On peut citer à titre d'exemples de polymères préférés, Carbopol ETD-2020 (copolymère acide acrylique / méthacrylate d'alkyle en $C_{10}$-$C_{30}$, réticulé - commercialisé par la société Noveon) ; Carbopol 1382, Pemulen TR1, Pemulen TR2 (copolymères acide acrylique/acrylate d'alkyle en $C_{10-30}$, réticulés - commercialisés par la société Noveon) le copolymère acide méthacrylique/acrylate d'éthyle/méthacrylate de stéaryle oxyéthyléné (55/35/10); le copolymère acide (méth)acrylique/acrylate d'éthyle/méthacrylate de béhényle oxyéthyléné 25 OE (Aculyn 28 commercialisé par Rohm & Haas) et le copolymère réticulé acide méthacrylique/acrylate d'éthyle/stéareth-10 allyl éther.

Lorsque la composition oxydante comprend un ou polymères amphiphiles à chaîne hydrophobe, alors celui-ci ou ceux-ci représentent généralement de 0,05 à 20 % en poids et, mieux encore, de 0,1 à 10 % du poids total de cette composition.

(C) <u>les agents tensioactifs</u> :

Le ou les agents tensioactifs susceptibles d'être présents dans la composition oxydante peuvent être indifféremment choisis parmi les tensioactifs anioniques, non ioniques, amphotères et cationiques.

Des agents tensioactifs anioniques, non ioniques, amphotères ou cationiques convenant à la mise en oeuvre de l'invention sont notamment les suivants :

• <u>tensioactifs anioniques</u> :

A titre d'exemples d'agents tensioactifs anioniques susceptibles d'être utilisés, seuls ou en mélanges, on peut citer les sels, en particulier les sels alcalins (sels de sodium, sels de magnésium, sels d'ammonium, sels d'amines, sels d'aminoalcools, ...) des composés suivants : alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, $\alpha$-oléfine-sulfonates, paraffine-sulfonates ; alkyl($C_6$-$C_{24}$) sulfosuccinates, alkyl ($C_6$-$C_{24}$) éthersulfosuccinates, alkyl ($C_6$-$C_{24}$) amidesulfosuccinates, alkyl ($C_6$-$C_{24}$) sulfoacétates ; acyl ($C_6$-$C_{24}$) sarcosinates et acyl ($C_6$-$C_{24}$)glutamates.

On peut aussi citer les esters d'alkyl($C_6$-$C_{24}$)polyglycosides carboxyliques tels que les alkylpolyglucoside citrates, les alkylpolyglucoside tartrates, les alkylpolyglucoside sulfosuccinates et les alkylpolyglucoside sulfosuccinamates ; les acyliséthionates et les N-acyltaurates, le radical alkyle ou acyle de tous ces composés comportant, de préférence, de 12 à 20 atomes de carbone, et le radical aryle désignant, de préférence, un groupement phényle ou benzyle.

Sont également utilisables les sels d'acides gras tels que les sels des acides oléique, ricinoléique, palmitique et stéarique, des acides d'huile de coprah ou d'huile de coprah hydrogénée ; les acyllactylates dont le radical acyle comporte de 8 à 20 atomes de carbone ; les acides d'alkyl D galactoside uroniques et leurs sels ; les acides alkyl ($C_6$-$C_{24}$) éther carboxyliques polyoxyalkylénés, les acides alkyl($C_6$-$C_{24}$)amido éther carboxyliques polyoxyalkylénés et leurs sels, en particulier ceux comportant de 2 à 50 groupes oxydes d'alkylène et, plus spécialement oxydes d'éthylène, et leurs mélanges.

• tensioactifs non ioniques :

Les agents tensioactifs non ioniques sont des composés bien connus en eux-mêmes (voir, par exemple, le "Handbook of Surfactants", M.R. PORTER, Ed. Blackie & Son, Glasgow and London, 1991, 116-178) et leur nature ne revêt pas, dans le cadre de la présente invention, de caractère critique.

Ainsi, utilisés seuls ou en mélanges, ils peuvent notamment être choisis parmi les alcools, les $\alpha$-diols, les alkylphénols polyéthoxylés et polypropoxylés ayant une chaîne grasse comportant, par exemple, de 8 à 18 atomes de carbone, le nombre de groupes oxydes d'éthylène ou oxydes de propylène pouvant être notamment de 2 à 50 ; les copolymères d'oxyde d'éthylène et de propylène, les condensats d'oxyde d'éthylène et de propylène sur des alcools gras, les amides gras polyéthoxylés ayant, de préférence, de 2 à 30 moles d'oxyde d'éthylène ; les amides gras polyglycérolés comportant en moyenne de 1 à 5 et, plus spécialement, de 1,5 à 4, groupes glycérol ; les esters d'acide gras du sorbitan oxyéthylénés comportant de 2 à 30 moles d'oxyde d'éthylène ; les esters d'acides gras du sucrose, les esters d'acides gras du polyéthylèneglycol ; les alkylpolyglycosides ; les dérivés de N-alkyl glucamine et les oxydes d'amines tels que les oxydes d'alkyl($C_{10}$-$C_{14}$) amines ou les oxydes de N-acylaminopropylmorpholine.

• tensioactifs amphotères :

Les agents tensioactifs amphotères (ou zwittérioniques), dont la nature ne revêt pas de caractère critique dans le cadre de la présente invention, peuvent notamment être choisis, seuls ou en mélanges, parmi les dérivés d'amines secondaires ou tertiaires aliphatiques dont le radical aliphatique est une chaîne linéaire ou ramifiée comportant de 8 à 18 atomes de carbone et contenant au moins un groupe anionique hydrosolubilisant, par exemple un carboxylate, un sulfonate, un sulfate, un phosphate ou un phosphonate.

On peut également citer les alkyl ($C_8$-$C_{20}$)bétaïnes, les sulfobétaïnes, les alkyl($C_8$-$C_{20}$)amidoalkyl ($C_1$-$C_6$) bétaïnes et les alkyl ($C_8$-$C_{20}$) amidoalkyl ($C_1$-$C_6$)sulfobétaïnes.

Parmi les dérivés d'amines, on peut notamment citer les composés commercialisés par la société Rhodia Chimie sous la dénomination commerciale Miranol® , qui sont décrits dans US 2,528,378 et US 2,781,354 et qui sont classés dans le Dictionnaire CTFA, 5ème édition, 1993, sous les désignations anglosaxonnes "disodium cocoamphodiacetate", "disodium lauroamphodiacetate", "disodium caprylamphodiacetate", "disodium capryloamphodiacetate", "disodium cocoamphodipropionate", "disodium lauroamphodipropionate", "disodium caprylamphodipropionate", "disodium capryloamphodipropionate", "lauroamphodiproponic acid" et "cocoamphodipropionic acid".

• tensioactifs cationiques :

Comme agents tensioactifs cationiques aptes à être utilisés seuls ou en mélanges, on peut citer les sels d'amines grasses primaires, secondaires et tertiaires, éventuellement polyoxyalkylénées ; les sels d'ammonium quaternaire tels que les chlorures et les bromures de tétraalkylammonium, d'alkylamidoalkyltrialkylammonium, de trialkylbenzylammonium, de trialkylhydroxyalkylammonium et d'alkylpyridinium ; les dérivés d'imidazoline et les oxydes d'amines à caractère cationique.

Lorsque la composition oxydante comprend un ou plusieurs agents tensioactifs, alors celui-ci ou ceux-ci représentent généralement de 0,01 à 40% en poids et, mieux encore, de 0,1 à 30% du poids total de cette composition.

(D) les agents d'ajustement de la rhéologie autres que les polymères (B) :

Au sens de la présente invention, on entend par "agent d'ajustement de la rhéologie", tout composé propre à conférer une viscosité à la composition oxydante telle qu'une fois appliquée sur des fibres kératiniques, cette dernière ne coule pas et reste parfaitement localisée au point d'application.

Notons que ledit agent décrit ci-après est dépourvu de chaîne hydrophobe, c'est-à-dire de chaîne hydrocarbonée, saturée ou non, aromatique ou non, linéaire ou ramifiée, en $C_8$-$C_{30}$, comprenant éventuellement un ou plusieurs motifs oxyalkylénés (oxyéthylénés et/ou oxypropylénés).

Le ou les agents d'ajustement de la rhéologie susceptibles d'être présents dans la composition oxydante sont des polymères d'origine naturelle ou les polymères synthétiques, et sont avantageusement choisis parmi ceux utilisés

classiquement dans le domaine cosmétique.

Comme exemples de polymères synthétiques, on peut citer, la polyvinylpyrrolidone, l'acide polyacrylique, le polyacrylamide, l'acide poly-2-acrylamidopropanesulfonique non réticulé (Simugel EG de la société SEPPIC), l'acide poly-2-acrylamido-2-méthylpropane sulfonique réticulé, libre ou partiellement neutralisé par l'ammoniaque (Hostacerin AMPS de Clariant), des mélanges d'acide poly-2-acrylamido-2-méthylpropane sulfonique non réticulé avec des éthers d'hydroxyalkylcellulose ou avec des poly(oxyde d'éthylène) tels que décrits dans le brevet US 4,540,510 ; des mélanges d'acide poly (méth) acrylamido-alkyl ($C_1$-$C_4$) -sulfonique, de préférence réticulé, avec un copolymère réticulé de l'anhydride maléique et d'un alkyl ($C_1$-$C_5$) vinyléther (Hostacerin AMPS / Stabileze QM de la société ISF).

Les polymères épaississants d'origine naturelle sont de préférence des polymères comportant au moins un motif sucre, comme les gommes de guar non ioniques, modifiées ou non par des groupements hydroxyalkyle en $C_1$-$C_6$ ; les gommes de biopoly saccharides d'origine microbienne telles que les gommes de scléroglucane ou de xanthane ; les gommes issues d'exudats végétaux telles que les gommes arabique, ghatti, karaya, tragacanthe, carraghénanne, agar et caroube ; les pectines ; les alginates ; les amidons ; les hydroxyalkyl($C_1$-$C_6$)celluloses et carboxyalkyl($C_1$-$C_6$) celluloses.

Notons que les termes "motif sucre" désignent une portion monosaccharidique (c'est-à-dire monosaccharide ou oside ou sucre simple), une portion oligosaccharidique (chaînes courtes formées de l'enchaînement d'unités monosaccha ridiques, éventuellement différentes) ou une portion polysaccharidique [longues chaînes constituées d'unités monosaccharidiques, éventuellement différentes, c'est-à-dire polyholosides ou polyosides]. Les unités saccharidiques peuvent être en outre substituées par des radicaux alkyle, ou hydroxyalkyle, ou alcoxy, ou acyloxy, ou carboxyle, les radicaux alkyle en $C_1$-$C_4$.

A titre d'exemples de gommes de guar non ioniques non modifiées, on peut citer entre autres Guargel D/15 (Goodrich) ; Vidogum GH 175 (Unipectine), Meypro-Guar 50 et Jaguar C (Meyhall/Rhodia Chimie) ; et à titre de gommes de guar non ioniques modifiées, Jaguar HP8, HP60, HP120, DC 293, HP 105 (Meyhall/Rhodia Chimie) ; Galactasol 4H4FD2 (Aqualon).

Les gommes de biopolysaccharides d'origine microbienne, végétale sont bien connues de l'homme de l'art et décrites notamment dans l'ouvrage de Robert L. Davidson intitulé "Handbook of Water soluble gums and resins" édité chez Mc Graw Hill Book Company (1980).

Parmi ces gommes, citons les scléroglucanes comme notamment Actigum CS de Sanofi Bio Industries ; Amigel de Alban Muller International, ainsi que les scléroglucanes traités au glyoxal décrits dans FR 2 633 940) ; les gommes xanthanes comme Keltrol, Keltrol T, Keltrol Tf, Keltrol Bt, Keltrol Rd, Keltrol Cg (Nutrasweet Kelco), Rhodicare S, Rhodicare H (Rhodia Chimie) ; les dérivés d'amidon comme Primogel (Avebe) ; les hydroxyéthylcelluloses telles que Cellosize QP3L, QP4400H, QP30000H, HEC30000A, Polymer PCG10 (Amerchol), Natrosol 250HHR, 250MR, 250M, 250HHXR, 250HHX, 250HR, HX (Hercules), Tylose H1000 (Hoechst) ; les hydroxypropylcelluloses comme Klucel EF, H, LHF, MF, G (Aqualon) ; les carboxyméthylcelluloses comme Blanose 7M8/SF, raffinée 7M, 7LF, 7MF, 9M31F, 12M31XP, 12M31P, 9M31XF, 7H, 7M31, 7H3SXF (Aqualon), Aquasorb A500 (Hercules), Ambergum 1221 (Hercules), Cellogen HP810A, HP6HS9 (Montello), Primellose (Avebe).

La composition peut, de plus comprendre, en remplacement ou en association avec au moins un agent d'ajustement de la rhéologie, au moins un alkylamide d'acide carboxylique en $C_6$-$C_{30}$, linéaire ou non, saturé ou non, et portant éventuellement un ou plusieurs groupements hydroxyles.

Par ailleurs, l'azote du groupement amide peut être mono- ou di-substitué. Il est de préférence monosubstitué.

L'amide peut comprendre 1 à 20 motifs oxyalkylénés (oxyéthylénés et/ou oxypropylénés), de préférence, oxyéthylénés.

Lorsque la composition oxydante comprend un ou plusieurs agents d'ajustement de la rhéologie, alors celui-ci ou ceux-ci représentent généralement de 0,05 à 20% en poids et, mieux encore, de 0,1 à 10 % du poids total de cette composition.

(E) <u>les agents d'ajustement du pH</u> :

Le pH de la composition oxydante peut être compris entre 1,5 et 12.

Toutefois, on préfère que ce pH soit compris entre 1,5 et 10 et, mieux encore, entre 1,5 et 7 dans le cas où la composition oxydante est destinée à la décoloration de fibres kératiniques, et qu'il soit compris entre 6 et 12 et, de préférence, entre 7 et 11 lorsqu'elle est destinée à la déformation permanente de fibres kératiniques.

De telles valeurs de pH peuvent être obtenues au moyen d'agents acidifiants ou alcalinisants.

A titre d'exemples d'agents acidifiants susceptibles d'être utilisés, on peut citer les acides minéraux ou organiques comme l'acide chlorhydrique, l'acide phosphorique, l'acide orthophosphorique, l'acide acétique, l'acide tartrique, l'acide citrique, l'acide lactique, l'acide borique, et les acides sulfoniques.

Les agents alcalinisants peuvent, eux, être notamment choisis parmi l'ammoniaque, les carbonates alcalins ou d'ammonium, les alcanolamines telles que les mono-, di-et triéthanolamines ainsi que leurs dérivés, les hydroxyalkylamines, les éthylène-diamines oxyéthylénées et/ou oxypropylénées, les hydroxydes de sodium ou de potassium et les composés de formule (XIX) suivante :

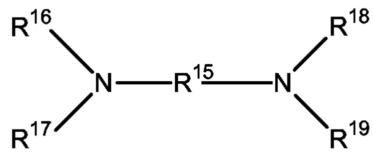

(XIX)

dans laquelle :

- $R^{15}$ est un reste propylène éventuellement substitué par un groupement hydroxyle ou un radical alkyle en $C_1$-$C_4$ ; tandis que
- $R^{16}$, $R^{17}$, $R^{18}$ et $R^{19}$, qui sont identiques ou différents, représentent un atome d'hydrogène, un radical alkyle en $C_1$-$C_4$ ou un radical hydroxyalkyle en $C_1$-$C_4$.

Lorsque la composition oxydante comprend un ou plusieurs agents acidifiants ou un ou plusieurs agents alcalinisants, alors celui-ci ou ceux-ci représentent généralement de 0,01 à 30% en poids du poids total de cette composition.

(F) <u>les solvants</u> :

Les solvants susceptibles d'être présents dans la composition oxydante sont notamment l'eau et des mélanges composés d'eau et d'un ou plusieurs solvants organiques acceptables sur le plan cosmétique, ce ou ces solvants organiques pouvant être en particulier des alcools tels que l'éthanol, l'isopropanol, l'alcool benzylique, l'alcool phényléthylique ou l'alcool cétylique ; des polyols comme le propylèneglycol et le glycérol, des éthers de glycols comme les éthers monométhylique, monoéthylique et monobutylique d'éthylèneglycol, ainsi que des alkyléthers de glycols comme le monoéthyléther ou le monobutyléther du diéthylèneglycol.
Ce ou ses solvants organiques, lorsqu'ils présents dans la composition oxydante, représentent généralement de 0,5 à 20% en poids et, mieux encore, de 2 à 10% en poids du poids total de cette composition.
Selon sa destination et les propriétés particulières que l'on souhaite lui conférer, la composition oxydante peut aussi comprendre un ou plusieurs additifs choisis parmi :

- les charges minérales ou organiques telles que la silice ou les argiles, et/ou
- les liants tels que la vinylpyrrolidone, les huiles ou les cires, les polyalkylèneglycols ou les dérivés de polyalkylèneglycols, et/ou
- les lubrifiants tels que les stéarates de polyols ou les stéarates de métaux alcalins ou alcalino-terreux, et/ou
- les agents antimousse, et/ou
- les silicones volatiles ou non, cycliques, linéaires ou ramifiées, et éventuellement modifiées, notamment par des groupements amines, et/ou
- les agents colorants, et/ou
- les agents matifiants comme les oxydes de titane, et/ou
- les conservateurs, et/ou
- les parfums.

Chacun de ces adjuvants peut représenter, lorsqu'il est présent dans la composition oxydante, jusqu'à 30% en poids du poids total de cette composition.
Conformément à l'invention, la composition oxydante est une composition qui est, de préférence, destinée à la coloration, la décoloration ou à la déformation permanente de fibres kératiniques humaines et, plus spécialement de cheveux.
La présente invention a également pour objet une composition oxydante pour la coloration, la décoloration ou la déformation permanente de fibres kératiniques, comprenant au moins un agent oxydant et au moins un composé répondant à la formule (I) suivante :

$$R\text{-}N\text{-}(CH(R')CO_2X)_2$$

(I)

dans laquelle :

- R représente un atome d'hydrogène ou un groupe CH $(CO_2X)$ $(CH_2)$ $_2CO_2X$, CH $(CH_3)$ $-CO_2X$ ou $(CH_2)$ $_2$-N(COR") $-CH_2-CO_2X$ ;
- R" représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique ou d'un cation ammonium ;

à la condition que, lorsque l'agent oxydant est le perborate de sodium, le composé de formule (I) est différent de l'acide méthylglycine diacétique et de l'acide iminodisuccinique.

Selon l'invention, lorsque X représente un cation métallique monovalent, celui peut être choisi dans le groupe des cations de métaux alcalins, tel $K^+$ et $Na^+$.

Selon l'invention, lorsque X représente un cation métallique bivalent, celui-ci peut être choisi dans le groupe des cations de métaux alcalino-terreux ou de métaux de transition, tel que $Ca^{2+}$, $Mg^{2+}$ , $Co^{2+}$, $Fe^{2+}$, $Mn^{2+}$ et $Zn^{2+}$.

Comme énoncé précédemment, parmi les composés de formule (I) ci-dessus susceptibles d'entrer dans la composition de l'invention, on peut citer les composés préférés choisis dans le groupe constitué par l'acide méthylglycine diacétique, l'acide N-lauroyl-N,N',N'-triacétique éthylènediamine, l'acide iminodisuccinique, l'acide N,N-dicarboxyméthyl L-glutamique, l'acide iminodisuccinique et leurs sels correspondants, , il le composé le plus préféré étant l'acide méthylglycinediacétique.

Comme énoncé précédemment, la composition oxydante, selon l'invention, comprend au moins un agent oxydant, ledit agent oxydant pouvant être choisi parmi le peroxyde d'hydrogène, le peroxyde d'urée, les bromates de métaux alcalins, les persels tels que les perborates, percarbonates et persulfates, les peracides. De préférence, l'agent oxydant est un persulfate.

Conformément à l'invention, on préfère les compositions dans lesquelles les composés de formule (I) sont l'acide méthylglycinediacétique ou l'acide iminodisuccinique, éventuellement sous forme de sels et l'agent oxydant est l'eau oxygénée.

Comme énoncé précédemment, le ou les composés de formule (I) représentent, de préférence, de 0,001 à 5% en poids du poids total de la composition oxydante.

Comme énoncé précédemment, la composition oxydante, selon l'invention, peut comprendre, en plus du ou des composés de formule (I) et du ou des agents oxydants, un ou plusieurs composés choisis parmi :

- les polymères substantifs cationiques ou amphotères (A) ;
- les polymères amphiphiles non ioniques, anioniques, cationiques ou amphotères, comportant au moins une chaîne grasse (B) ;
- les agents tensioactifs anioniques, non-ioniques, cationiques, amphotères ou zwittérioniques (C) ;
- les agents d'ajustement de la rhéologie (D) autres que les polymères (B) ;
- les agents d'ajustement du pH (E) ;
- les solvants (F).
- d'autres adjuvants ;

Ces composés peuvent être choisis parmi ceux énoncés précédemment et sont, présents, dans la composition oxydante, dans des proportions analogues à celles précédemment indiquées pour chacun d'eux.

La présente invention a également pour objet un procédé de décoloration ou de déformation permanente de fibres kératiniques, comprenant successivement les étapes consistant à :

a) appliquer sur les fibres kératiniques une composition oxydante telle que définie précédemment ;
b) laisser reposer la composition oxydante sur les fibres kératiniques pendant un temps suffisant pour obtenir la décoloration ou la déformation recherchée ;
c) rincer les fibres kératiniques pour en éliminer la composition oxydante ;
d) laver éventuellement les fibres kératiniques une ou plusieurs fois, les rincer après chaque lavage et éventuellement, les sécher ;

ledit procédé comprenant de plus, avant l'étape a), dans le cas d'une déformation permanente, les étapes consistant à :

i) appliquer sur les fibres kératiniques une composition réductrice, lesdites fibres kératiniques étant mis sous tension mécanique avant, pendant, ou après ladite application ;
ii) laisser reposer la composition réductrice sur les fibres kératiniques pendant un temps suffisant pour obtenir la déformation recherchée ; et
iii) rincer éventuellement les fibres kératiniques à l'eau pour en éliminer la composition réductrice.

Lorsque ce procédé est un procédé de déformation permanente, la première étape de ce procédé consiste à appliquer sur les cheveux une composition réductrice. Cette application se fait mèche par mèche ou globalement.
La composition réductrice comprend au moins un agent réducteur, qui peut être en particulier choisi parmi l'acide thioglycolique, la cystéine, la cystéamine, le thioglycolate de glycérol, l'acide thiolactique, ou les sels de l'acide thiolactique ou thioglycolique.
L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.
Avant de procéder à l'étape (iii) facultative de rinçage, il convient de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35°C à 45°C, en protégeant de préférence, également les cheveux par un bonnet.
Dans l'étape facultative de rinçage, les cheveux imprégnés de la composition réductrice sont donc ensuite rincés soigneusement par une composition aqueuse.
Puis, après l'étape éventuelle de rinçage, on applique sur les cheveux la composition oxydante de l'invention (étape a)), dans le but de fixer la nouvelle forme imposée aux cheveux.
Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.
Si la tension des cheveux est maintenue par des moyens extérieurs, on peut retirer de la chevelure ces derniers (rouleaux, bigoudis et analogues) avant ou après l'étape de fixation.
Enfin, dans la dernière étape du procédé selon l'invention (étape c), les cheveux imprégnés de la composition oxydante sont rincés soigneusement, généralement à l'eau.
Lorsque le procédé est une procédé de décoloration de fibres kératiniques, ledit procédé comprend tel que défini précédemment, une étape d'application sur les fibres kératiniques d'une composition oxydante selon l'invention. Classiquement, une deuxième étape du procédé de décoloration selon l'invention est une étape de rinçage des fibres kératiniques.
La présente invention a également pour objet un procédé de coloration de fibres kératiniques comprenant successivement les étapes consistant à :

e) appliquer sur ces fibres une composition tinctoriale ;
f) révéler la couleur de ladite composition par application sur les fibres d'une composition oxydante telle que définie précédemment;
g) laisser reposer la composition oxydante sur les fibres kératiniques pendant un temps suffisant pour obtenir la coloration recherchée ;
h) rincer les fibres kératiniques à l'eau pour en éliminer la composition tinctoriale et la composition oxydante.

Selon une autre variante de l'invention, le procédé comprend successivement les étapes consistant à :

i) appliquer sur les fibres kératiniques une composition obtenue par mélange extemporané avant application, d'une composition tinctoriale et d'une composition oxydante telle que décrite auparavant ;
j) laisser reposer la composition sur les fibres kératiniques pendant un temps suffisant pour obtenir la coloration recherchée ;
k) rincer les fibres kératiniques à l'eau pour en éliminer la composition.

Selon une autre forme de réalisation particulière de l'invention, le procédé de coloration peut comporter une étape préliminaire consistant à mélanger, avant application sur les fibres kératiniques, une composition comprenant

au moins un précurseur de colorant avec une composition oxydante selon l'invention, cette dernière permettant grâce à la présence d'un agent oxydant et à la présence d'un agent complexant conforme à l'invention, la révélation du précurseur de colorant de la première composition, ladite composition résultante étant ensuite appliquée sur les fibres kératiniques.

Quel que soit le mode de réalisation de ce procédé de coloration, le temps nécessaire à la révélation s'échelonne généralement entre 3 et 60 minutes, plus précisément entre 5 et 40 minutes, le temps de repos après application des compositions sur les fibres kératiniques s'échelonnant de 5 minutes à une heure, de préférence de 10 à 30 minutes.

Un autre objet de la présente invention est un dispositif ou « kit » pour la coloration de fibres kératiniques, comprenant au moins deux compositions A et B destinées à être mélangées ensemble pour obtenir une composition colorante prête à l'emploi, la composition A étant la composition oxydante et la composition B étant une composition comprenant au moins un colorant , ledit dispositif étant caractérisé en ce que la composition A contient au moins un ou plusieurs composés répondant à la formule générale (I) suivante :

$$R\text{-}N\text{-}(CH(R')CO_2X)_2$$

(I)

dans laquelle :

- R représente un atome d'hydrogène ou un groupe CH $(CO_2X)$ $(CH_2)$ $_2CO_2X$, CH $(CH_3)$ -$CO_2X$ ou $(CH_2)_2$-N(COR") -$CH_2$-$CO_2X$ ;
- R" représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium, à la condition que, lorsque l'agent oxydant est le perborate de sodium, le composé de formule (I) est différent de l'acide méthylglycine diacétique et de l'acide iminodisuccinique.

L'invention a également pour objet un dispositif ou « kit » pour la décoloration de fibres kératiniques, comprenant au moins deux compositions C et D destinées à être mélangées ensemble pour obtenir une composition oxydante prête à l'emploi, ledit dispositif étant caractérisé en ce l'une au moins des compositions C et D contient un ou plusieurs agents oxydants et l'une au moins contient un ou plusieurs composés répondant à la formule générale (I) suivante :

$$R\text{-}N\text{-}(CH(R')CO_2X)_2$$

(I)

dans laquelle :

- R représente un atome d'hydrogène ou un groupe CH $(CO_2X)$ $(CH_2)$ $_2CO_2X$, CH $(CH_3)$ -$CO_2X$ ou $(CH_2)$ $_2$-N(COR") -$CH_2$-$CO_2X$ ;
- R" représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium, à la condition que, lorsque l'agent oxydant est le perborate de sodium, le composé de formule (I) est différent de l'acide méthylglycine diacétique et de l'acide iminodisuccinique.

Enfin, l'invention a pour objet un dispositif ou « kit » pour la déformation permanente des fibres kératiniques, comprenant au moins deux compositions E et F ; la composition E étant une composition oxydante et la composition F une composition réductrice, ledit dispositif étant caractérisé en ce que la composition E contient un ou plusieurs

agents oxydants et au moins un ou plusieurs composés répondant à la formule générale (I) suivante :

$$R\text{-}N\text{-}(CH(R')CO_2X)_2$$

(I)

dans laquelle :

- R représente un atome d'hydrogène ou un groupe CH $(CO_2X)$- $(CH_2)$ $_2CO_2X$, CH $(CH_3)$ -$CO_2X$ ou $(CH_2)$ $_2$-N(COR") -$CH_2$-$CO_2X$ ;
- R" représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium, à la condition que, lorsque l'agent oxydant est le perborate de sodium, le composé de formule (I) est différent de l'acide méthylglycine diacétique et de l'acide iminodisuccinique.

L'invention a enfin pour objet l'utilisation d'une composition telle que définie précédemment, ou d'un procédé tel que défini précédemment ou d'un dispositif tel que défini précédemment pour la coloration, la décoloration ou la déformation permanente des fibres kératiniques humaines et, plus spécialement, des cheveux.

Outre les dispositions qui précèdent, l'invention comprend encore d'autres dispositions qui ressortiront du complément de la description qui suit, qui se réfère à des exemples de réalisation de compositions réductrices pour la coloration, la décoloration, et pour la déformation permanente de fibres kératiniques.

Il va de soi que ces exemples sont donnés à titre illustratif et en aucun cas limitatif de l'objet de l'invention.

## EXPOSÉ DÉTAILLÉ DE MODES DE RÉALISATION PARTICULIERS

### EXEMPLE 1

Dans cet exemple, on teste la stabilité de deux compositions oxydantes -respectivement A et B-, la composition oxydante A étant une composition comprenant un agent complexant non conforme à l'invention (l'acide diéthylène triamine pentaacétique sous forme de sel) et la composition B étant une composition comprenant un agent complexant conforme à la présente invention (l'acide méthylglycinediacétique sous forme de sel). Le tableau I ci-dessous spécifie les constituants entrant dans les compositions A et B, les quantités étant exprimées en pourcentage massique.

TABLEAU I

| Constituants | Composition A | Composition B |
|---|---|---|
| Acide diéthylène triamine pentacétique sous forme de sel pentasodique en solution aqueuse à 40% (1) | 0,176 (soit 0,14 mole) | - |
| Acide méthylglycinediac étique sous forme de sel trisodique, en solution aqueuse à 40% (2) | - | 0,075 (soit 0,14 mole) |
| Eau oxygénée à 50% | 12 | 12 |
| Acide phosphorique à 85% | q.s.p pH=2 | q.s.p pH=2 |
| Eau | q.s.p 100g | q.s.p 100g |

(1)Dissolvine®- Société Azko
(2)Trilon M Liquide ®-Société BASF

La stabilité de ces deux compositions est déterminée en mesurant le titre en eau oxygénée, avant (t1) et après (t2).

L'indice de stabilité correspond au rapport du titre en eau oxygénée à t2 sur le titre en eau oxygénée à t1.

Trois essais ont été réalisés pour chaque composition. Le tableau II regroupe les indices de stabilité moyens pour chacune des compositions susmentionnées.

TABLEAU II

| | Composition A | Composition B |
|---|---|---|
| Stabilité des compositions oxydantes<br>Taux de $H_2O_2$ résiduel : - Moyenne - (Ecart-type)<br>Volume $O_2$ libéré pour 100 g de composition : | <br>96,4 % (0,7 %)<br>151 ml | <br>99,4% (0,5%)<br>23 ml |

EXEMPLE 2

On a préparé, dans cet exemple, deux compositions oxydantes-respectivement C et D destinées à un usage pour la coloration de fibres kératiniques, la composition C étant une composition comprenant un agent complexant non conforme à l'invention et la composition D étant une composition comprenant un agent complexant conforme à l'invention.

Le tableau III représente les compositions qualitatives et quantitatives de ces compositions, les quantités étant exprimées en pourcentage massique.

TABLEAU III

| Constituants | Composition C | Composition D |
|---|---|---|
| Acide diéthylène triamine pentacétique sous forme de sel pentasodique en solution aqueuse à 40% (9) | 0,150<br>(soit 0,12 mmole) | - |
| Acide méthylglycinediac étique, sous forme de sel trisodique en solution aqueuse à 40% (10) | - | 0,065<br>(soit 0,12 mmole) |
| Salicylate de sodium | 0,024 | 0,024 |
| Pyrophosphate tétrasodique, $10H_2O$ | 0,01 | 0,01 |
| Eau oxygénée à 50% | 12 | 12 |
| Acide phosphorique à 85% | q.s.p pH=2 | q.s.p pH=2 |
| Eau | q.s.p 100g | q.s.p 100g |

(9)Dissolvine®- Société Azko
(10) Trilon M Liquide ®-Société BASF

Les compositions C et D ont été mélangées avec une quantité égale d'une composition colorante neutre Recital dans laquelle on introduit :

- $10^{-3}$ moles de p-aminophénol, et
- $10^{-3}$ moles de 4-amino-2-hydroxytoluène.

Les compositions résultantes sont appliquées pendant 30 minutes sur des mèches de cheveux gris naturels permanentées à 90% de blancs, ceci dans un rapport de 10 g de composition pour 1 g de mèche de cheveux.

EXEMPLE 3

On a préparé, dans cet exemple, une composition oxydante E- destinée à un usage pour la déformation permanente de fibres kératiniques, et comprenant un agent complexant conforme à l'invention.

Le tableau IV en donne la composition qualitative et quantitative, les quantités étant exprimées en pourcentage massique.

TABLEAU IV

| Constituants | Composition E |
|---|---|
| Alcool cétylique | 3 |
| Lauryl sulfate de sodium | 0,5 |
| Alcool oléique polyglycérolé (2 moles) | 0,45 |

TABLEAU IV (suite)

| Constituants | Composition E |
|---|---|
| Alcool oléique polyglycérolé (4 moles) | 0,35 |
| Siméthicone | 0,045 |
| Acide méthylglycine diacétique sous forme de sel trisodique en solution aqueuse à 40% | 0,13 |
| | |
| Pyrophosphate tétrasodique, $10H_2O$ | 0,02 |
| Salicylate de sodium | - |
| Stannate de sodium, $6H_2O$ | 0,04 |
| Eau oxygénée à 50% | 24 |
| Acide phosphorique à 85% en solution aqueuse | q.s.p pH=2 |
| Eau | q.s.p 100g |

EXEMPLE 4

On a préparé, dans cet exemple, deux compositions oxydantes -respectivement F et G-destinées à un usage pour la décoloration, ces compositions étant toutes deux des compositions anhydres, sous forme pulvérulente, comprenant un agent complexant conforme à l'invention.

Le tableau V représente les compositions qualitatives et quantitatives de ces compositions, les quantités étant exprimées en pourcentage massique.

TABLEAU V

| Constituants | Composition F | Composition G |
|---|---|---|
| Persulfate de potassium | 39,5 | 46 |
| Persulfate de sodium | 30 | 15 |
| Disilicate de sodium | - | 15 |
| Métasilicate de sodium | 14 | 4 |
| Chlorure d'ammonium | 6 | 4 |
| Urée | - | 4,5 |
| Imino disuccinate de sodium(13) | 1 | 0,8 |
| Copolymère hexaméthyl diisocyanate/polyéthylèn eglycol à terminaison $\alpha$ et $\omega$ stéaryl polyoxyéthylène(14) | 0,5 | - |
| Copolymère acide acrylique/méthacrylate d'alkyl (C10/C30) réticulé (15) | - | 1 |
| Carboxyméthyl amidon de pommme de terre/sel de sodium faiblement réticulé | - | 2 |
| Gomme de Guar | 2 | 1,5 |
| Colorant (ultramarine) | 0,5 | - |
| Oxyde de titane | 0,5 | 1 |
| Lauryl sulfate de sodium | 2 | 2 |
| Stéarate de calcium | 1 | 1 |
| Silice pyrogénée à caractère hydrophile | 3 | 0,2 |

(13) Imino dissucinate VP OC sodium salt powder (N-305) - Société BAYER ;
(14) SER-AD FX 1100® -Société SERVO DELDEN ;
(15)CARBOPOL ETD 2020® -Société NOVEON ;

TABLEAU V (suite)

| Constituants | Composition F | Composition G |
|---|---|---|
| Polydécène hydrogéné (16) | - | 2 |

(16)SILKFLO 366 NE POLYDECECE® -Société AMOCO CHEMICAL

La composition de décoloration F (40g) est mélangée avec la composition oxydante à base d'eau oxygénée E (80 g). On applique le mélange décolorant prêt à l'emploi ainsi obtenu 45 minutes sous casque sur cheveux naturels foncés, puis on rince abondamment à l'eau. A l'issue de ces opérations, on obtient une décoloration puissante et homogène.

La composition de décoloration G (40g) est mélangée avec la composition oxydante à base d'eau oxygénée E (60g). On applique le mélange décolorant prêt à l'emploi ainsi obtenu 30 minutes sous casque sur cheveux naturels foncés, puis on rince abondamment à l'eau. A l'issue de ces opérations, on obtient une décoloration puissante et homogène, avec des cheveux doux et brillants et faciles à démêler.

EXEMPLE 5

On a préparé, dans cet exemple, deux compositions oxydantes -respectivement H et I-destinées à un usage pour la décoloration, ces compositions étant toutes deux des compositions anhydres, sous forme de pâte, comprenant un agent complexant conforme à l'invention.

Le tableau VI représente les compositions qualitatives et quantitatives de ces compositions, les quantités étant exprimées en pourcentage massique.

TABLEAU VI

| Constituants | Composition H | Composition I |
|---|---|---|
| Persulfate de potassium | 35,8 | 35,6 |
| Persulfate de sodium | 6 | 6 |
| Disilicate de sodium | 15 | 15 |
| Métasilicate de sodium | 3 | 3 |
| Chlorure d'ammonium | 4,2 | 4,2 |
| Imino disuccinate de sodium(17) | 1 | 1 |
| Copolymère hexaméthyl diisocyanate/polyéthyl èneglycol à terminaison $\alpha$ et $\omega$ stéaryl polyoxyéthylène(18) | 2 | 0,5 |
| Copolymère acide acrylique/méthacrylate d'alkyl (C10/C30) réticulé(19) | - | 0,5 |
| Carboxyméthyl amidon de pommme de terre/sel de sodium faiblement réticulé | 2 | 1 |
| Gomme de Guar | - | 2 |
| Colorant (ultramarine) | 0,5 | 0,5 |
| Oxyde de titane | 1 | 1 |
| Lauryl sulfate de sodium | 3,5 | 3,5 |
| Stéarate de calcium | 2 | 2 |
| Silice pyrogénée à caractère hydrophile | 0,5 | 0,5 |
| Palmitate d'isopropyle | 22,5 | - |
| Cire d'abeille | 1 | - |
| Polydécène hydrogéné | - | 23 |
| Silice pyrogénée à caractère hydrophobe | - | 0,7 |

(17) Imino dissucinate VP OC sodium salt powder (N-305)- Société BAYER ;
(18)SER-AD FX 1100® -Société SERVO DELDEN ;
(19)CARBOPOL ETD 2020® -Société NOVEON.

La composition de décoloration H (40g) est mélangée avec la composition oxydante à base d'eau oxygénée E (80 g). On applique le mélange décolorant prêt à l'emploi ainsi obtenu 45 minutes sous casque sur cheveux naturels foncés, puis on rince abondamment à l'eau. A l'issue de ces opérations, on obtient une décoloration puissante et homogène, avec des cheveux doux et brillants, et faciles à démêler.

La composition de décoloration I (40g) est mélangée avec la composition oxydante à base d'eau oxygénée E (60 g). On applique le mélange décolorant prêt à l'emploi ainsi obtenu 25 minutes sous casque sur cheveux naturels foncés, puis on rince abondamment à l'eau. A l'issue de ces opérations, on obtient une décoloration puissante et homogène, avec des cheveux doux et brillants, et faciles à démêler.

EXEMPLE 6

Dans cet exemple, dans un premier temps, on prépare une composition réductrice.

Le tableau VII donne la composition qualitative et quantitative, les quantités étant exprimées en pourcentage massique.

TABLEAU VII

| Constituants | Composition J |
|---|---|
| Acide thioglycolique | 9,2 |
| Arginine | 15 |
| Ammoniaque à 20% | 1,86 |
| Carbonate d'ammonium | 4,5 |
| Cocoylamidopropylbétaïne/monolaurate de glycérol (25/5) en solution aqueuse à 30% | 1,3 |
| Peptisant | 0,8 |
| Alcool isostéarylique | 12 |
| Agent complexant | 0,4 |
| Parfum | 0,4 |
| Eau | q.s.p 100g |

La composition J est appliquée sur une mèche de cheveux humides, préalablement enroulée sur un bigoudi de 9 mm de diamètre, le temps de pose étant de 10 minutes. Puis, on rince la mèche ainsi traitée abondamment à l'eau.

Dans un deuxième temps, on prépare une composition oxydante, dite composition K.
Le tableau VIII représente la composition qualitative et quantitative, les quantités étant exprimées en pourcentage massique.

TABLEAU VIII

| Constituants | Composition K |
|---|---|
| Alcool cétylique | 3 |
| Lauryl sulfate de sodium | 0,5 |
| Alcool oléique polyglycérolé (2 moles) | 0,45 |
| Alcool oléique polglycérolé (4 moles) | 0,35 |
| Siméthicone | 0,045 |
| Iminodisuccinate de sodium | 0,05 |
| Pyrophosphate tétrasodique, $10H_2O$ | 0,02 |
| Salicylate de sodium | - |
| Stannate de sodium, $6H_2O$ | 0,04 |
| Eau oxygénée à 50% | 5,4 |
| Acide phosphorique à 85% en solution aqueuse | q.s.p pH=2 |

TABLEAU VIII (suite)

| Constituants | Composition K |
|---|---|
| Eau | q.s.p 100g |
| [20]XUS-40855.00® -Société DOW CHEMICAL | |

La composition K est appliquée sur les mèches précédemment traitées par la composition réductrice J, par application de ladite composition K pendant un temps de pose de 10 minutes. Ensuite, les mèches sont rincées abondamment à l'eau. Enfin, les cheveux sont déroulés du bigoudi, puis séchés. Les mèches ainsi traitées sont ondulées.

## Revendications

1. Utilisation d'au moins un composé répondant à la formule (I) suivante :

$$R\text{-}N\text{-}(CH(R')CO_2X)_2$$

(I)

dans laquelle :

- R représente un atome d'hydrogène ou un groupe $CH(CO_2X)\text{-}(CH_2)_2CO_2X$, $CH(CH_3)\text{-}CO_2X$ ou $(CH_2)_2\text{-}N(COR'')\text{-}CH_2\text{-}CO_2X$ ;
- R'' représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium ;

en tant qu'agents complexants de cations métalliques présents dans une composition oxydante, comprenant au moins un oxydant, pour la décoloration, la coloration ou la déformation permanente de fibres kératiniques.

2. Utilisation selon la revendication 1, dans laquelle le cation monovalent ou divalent est, de préférence, choisi dans le groupe constitué par les cations monovalents de métaux alcalins, les cations divalents de métaux alcalino-terreux, les cations divalents de métaux de transition et les cations monovalents issus d'amines organiques ou d'ammonium.

3. Utilisation selon la revendication 1, dans laquelle le cation divalent est choisi dans le groupe des cations de métaux alcalino-terreux ou de métaux de transition.

4. Utilisation selon l'une quelconque des revendications 1 à 3, dans laquelle l'oxydant est un oxydant choisi dans le groupe constitué par le peroxyde d'hydrogène, les bromates de métaux alcalins, les persels.

5. Utilisation selon l'une quelconque des revendications 1 à 4, dans laquelle l'agent complexant est un agent choisi dans le groupe constitué par l'acide méthylglycine diacétique, l'acide N-lauroyl-N,N',N'-triacétique éthylènediamine, l'acide iminodisuccinique, l'acide N,N-dicarboxyméthyl L-glutamique et leurs sels correspondants.

6. Utilisation selon l'une quelconque des revendications précédentes, dans laquelle l'oxydant est l'eau oxygénée et le composé de formule (I) est l'acide méthylglycine diacétique, éventuellement sous forme de sels.

7. Utilisation selon l'une quelconque des revendications 1 à 6, dans laquelle l'oxydant est l'eau oxygénée et le composé de formule (I) est l'acide iminodisuccinique éventuellement sous forme de sels.

8. Utilisation selon l'une quelconque des revendications précédentes, dans laquelle la composition comprend, de plus, un ou plusieurs polymères conditionneurs cationiques ou amphotères, dans des proportions de 0,01 à 10%

en poids et, de préférence, de 0,05 à 5% en poids du poids total de ladite composition.

**9.** Utilisation selon l'une quelconque des revendications précédentes, dans laquelle la composition comprend, de plus, un ou plusieurs polymères amphiphiles non ioniques, anioniques, cationiques ou amphotères, comportant une chaîne hydrophobe, dans des proportions de 0,05 à 20 % en poids et, de préférence, de 0,1 à 10 % en poids du poids total de ladite composition.

**10.** Utilisation selon l'une quelconque des revendications précédentes, dans laquelle la composition comprend, de plus, un ou plusieurs agents tensioactifs, dans des proportions de 0,01 à 40% en poids et, de préférence, de 0,1 à 30% en poids du poids total de ladite composition.

**11.** Utilisation selon l'une quelconque des revendications précédentes, dans laquelle la composition comprend, de plus, un ou plusieurs agents d'ajustement de la rhéologie différents des polymères amphiphiles non ioniques, anioniques, cationiques ou amphotères, comportant une chaîne hydrophobe, dans des proportions de 0,05 à 20 % en poids et, de préférence, de 0,1 à 10 % en poids du poids total de ladite composition.

**12.** Utilisation selon l'une quelconque des revendications précédentes, dans laquelle la composition comprend, de plus, un ou plusieurs agents acidifiants ou alcalinisants, dans des proportions de 0,01 à 30% en poids du poids total de ladite composition.

**13.** Utilisation selon l'une quelconque des revendications précédentes, dans laquelle la composition comprend, de plus, un ou plusieurs solvants choisis dans le groupe constitué par l'eau et les mélanges composés d'eau et d'un ou plusieurs solvants organiques acceptables sur le plan cosmétique, ce ou ces solvants représentant de 0,5 à 20% en poids et, de préférence, de 2 à 10% en poids du poids total de ladite composition.

**14.** Utilisation selon l'une quelconque des revendications précédentes, dans laquelle la composition comprend, de plus, un ou plusieurs adjuvants choisis dans le groupe constitué par les charges minérales ou organiques, les liants, les lubrifiants, les agents antimousse, les silicones, les agents colorants, les agents matifiants, les conservateurs et les parfums.

**15.** Composition oxydante pour la coloration, la décoloration ou la déformation permanente de fibres kératiniques, comprenant au moins un oxydant et au moins un composé répondant à la formule (I) suivante :

$$R\text{-}N\text{-}(CH(R')CO_2X)_2$$

(I)

dans laquelle :

- R représente un atome d'hydrogène ou un groupe CH $(CO_2X)$ - $(CH_2)_2CO_2X$, CH $(CH_3)$ $-CO_2X$ ou $(CH_2)_2$-N (COR")-$CH_2$-$CO_2X$ ;
- R" représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium ;

à la condition que, lorsque l'agent oxydant est le perborate de sodium, le composé de formule (I) est différent de l'acide méthylglycine diacétique et de l'acide iminodisuccinique.

**16.** Composition selon la revendication 15, dans laquelle le cation monovalent est choisi dans le groupe des cations de métaux alcalins.

**17.** Composition selon la revendication 15, laquelle le cation divalent est choisi dans le groupe des cations de métaux alcalino-terreux ou de métaux de transition.

**18.** Composition selon l'une quelconque des revendications 15 à 17, dans laquelle l'oxydant est un oxydant choisi dans le groupe constitué par le peroxyde d'hydrogène, les bromates de métaux alcalins, les persels.

**19.** Composition selon la revendication 18, dans laquelle l'oxydant est un persulfate.

**20.** Composition selon l'une quelconque des revendications 15 à 19, dans laquelle le composé de formule (I) est un composé choisi dans le groupe constitué par l'acide méthylglycinediacétique, l'acide N-lauroyl-N,N',N'-triacétique éthylènediamine, l'acide N,N-dicarboxyméthyl L-glutamique, l'acide iminodisuccinique et leurs sels.

**21.** Composition selon l'une quelconque des revendications 15 à 20, dans laquelle l'oxydant est l'eau oxygénée et le composé de formule (I) est l'acide méthylglycinediacétique, éventuellement sous forme de sels.

**22.** Composition selon l'une quelconque des revendications 15 à 21, **caractérisée en ce qu'**elle comprend, de plus, un ou plusieurs constituants choisis dans le groupe constitué par les polymères conditionneurs cationiques ou amphotères, les polymères amphiphiles non ioniques, anioniques, cationiques ou amphotères, comportant une chaîne hydrophobe, les agents tensioactifs, les agents d'ajustement de la rhéologie différents des polymères amphiphiles précités, les agents d'ajustement du pH et les solvants.

**23.** Composition selon l'une quelconque des revendications 15 à 22, **caractérisée en ce qu'**elle comprend, de plus, un ou plusieurs adjuvants choisis dans le groupe constitué par les charges minérales ou organiques, les liants, les lubrifiants, les agents antimousse, les silicones, les agents colorants, les agents matifiants, les conservateurs et les parfums.

**24.** Procédé de décoloration ou de déformation permanente de fibres kératiniques, comprenant successivement les étapes consistant à :

a) appliquer sur les fibres kératiniques une composition oxydante telle que définie dans les revendications 15 à 23 ;
b) laisser reposer la composition oxydante sur les fibres kératiniques pendant un temps suffisant pour obtenir la décoloration ou la déformation recherchée ;
c) rincer les fibres kératiniques pour en éliminer la composition oxydante ;
d) laver les fibres kératiniques une ou plusieurs fois, les rincer après chaque lavage et éventuellement, les sécher ;

ledit procédé comprenant de plus, avant l'étape a), dans le cas d'une déformation permanente, les étapes consistant à :

i) appliquer sur les fibres kératiniques une composition réductrice ;
ii) laisser reposer la composition réductrice sur les fibres kératiniques pendant un temps suffisant pour obtenir la fixation de la déformation recherchée ; et
iii) rincer les fibres kératiniques à l'eau pour en éliminer la composition réductrice.

**25.** Procédé de coloration de fibres kératiniques comprenant successivement les étapes consistant à :

e) appliquer sur ces fibres une composition tinctoriale ;
f) révéler la couleur de ladite composition par application sur les fibres d'une composition oxydante selon l'une quelconque des revendications 15 à 23 ;
g) laisser reposer la composition oxydante sur les fibres kératiniques pendant un temps suffisant pour obtenir la coloration recherchée ;
h) rincer les fibres kératiniques à l'eau pour en éliminer la composition tinctoriale et la composition oxydante.

**26.** Procédé de coloration de fibres kératiniques comprenant successivement les étapes consistant à :

i) appliquer sur ces fibres une composition obtenue par mélange extemporané avant application d'une composition tinctoriale et d'une composition oxydante selon l'une quelconque des revendications 15 à 23 ;
j) laisser reposer la composition sur les fibres kératiniques pendant un temps suffisant pour obtenir la coloration recherchée ;
k) rincer les fibres kératiniques à l'eau pour en éliminer la composition.

**27.** Dispositif ou « kit » pour la coloration de fibres kératiniques, comprenant au moins deux compositions A et B destinées à être mélangées ensemble pour obtenir une composition colorante prête à l'emploi, la composition A étant la composition oxydante et la composition B étant une composition comprenant au moins un colorant, ledit dispositif étant **caractérisé en ce que** la composition A contient au moins un ou plusieurs composés répondant à la formule générale (I) suivante :

$$R\text{-}N\text{-}(CH(R')CO_2X)_2$$

(I)

dans laquelle :

- R représente un atome d'hydrogène ou un groupe $CH(CO_2X)\text{-}(CH_2)_2CO_2X$, $CH(CH_3)\text{-}CO_2X$ ou $(CH_2)_2\text{-}N(COR'')\text{-}CH_2\text{-}CO_2X$ ;
- R'' représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium,

à la condition que, lorsque l'agent oxydant est le perborate de sodium, le composé de formule (I) est différent de l'acide méthylglycine diacétique et de l'acide iminodisuccinique.

**28.** Dispositif ou « kit » pour la décoloration de fibres kératiniques, comprenant au moins deux compositions C et D destinées à être mélangées ensemble pour obtenir une composition oxydante prête à l'emploi, ledit dispositif étant **caractérisé en ce** l'une au moins des compositions C et D contient un ou plusieurs agents oxydants et l'une au moins contient un ou plusieurs composés répondant à la formule générale (I) suivante :

$$R\text{-}N\text{-}(CH(R')CO_2X)_2$$

(I)

dans laquelle :

- R représente un atome d'hydrogène ou un groupe $CH(CO_2X)\text{-}(CH_2)_2CO_2X$, $CH(CH_3)\text{-}CO_2X$ ou $(CH_2)_2\text{-}N(COR'')\text{-}CH_2\text{-}CO_2X$ ;
- R'' représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium, à la condition que, lorsque l'agent oxydant est le perborate de sodium, le composé de formule (I) est différent de l'acide méthylglycine diacétique et de l'acide iminodisuccinique.

**29.** Dispositif ou « kit » pour la déformation permanente des fibres kératiniques, comprenant au moins deux compositions E et F, la composition E étant une composition oxydante et la composition F une composition réductrice, ledit dispositif étant **caractérisé en ce que** la composition E contient un ou plusieurs agents oxydants et au moins un ou plusieurs composés répondant à la formule générale (I) suivante :

$$R\text{-}N\text{-}(CH(R')CO_2X)_2$$

(I)

dans laquelle :

- R représente un atome d'hydrogène ou un groupe $CH(CO_2X)-(CH_2)_2CO_2X$, $CH(CH_3)-CO_2X$ ou $(CH_2)_2-N(COR'')-CH_2-CO_2X$ ;
- R" représente un groupe alkyle, linéaire ou ramifié, comportant de 1 à 30 atomes de carbone, ou bien un groupe alkyle cyclique comportant de 3 à 30 atomes de carbone ;
- R' représente un groupe $CH_2CO_2X$ si R représente un atome d'hydrogène ou R' représente un atome d'hydrogène si R est différent d'un atome d'hydrogène ; et
- X représente un atome d'hydrogène ou un cation monovalent ou divalent issu d'un métal alcalin, d'un métal alcalino-terreux, d'un métal de transition, d'une amine organique, ou un cation ammonium,

à la condition que, lorsque l'agent oxydant est le perborate de sodium, le composé de formule (I) est différent de l'acide méthylglycine diacétique et de l'acide iminodisuccinique.

**30.** Utilisation d'une composition selon l'une quelconque des revendications 15 à 23, ou d'un procédé selon l'une des revendications 24 à 26 ou d'un dispositif selon la revendication 27, la revendication 28 ou la revendication 29 pour la coloration, la décoloration ou la déformation permanente des fibres kératiniques humaines et, plus spécialement, des cheveux.

Office européen des brevets

# RAPPORT DE RECHERCHE EUROPEENNE

Numéro de la demande
EP 04 10 1243

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | WO 03/015732 A (OREAL ;CANNELL DAVID W (US); NGUYEN NGHI VAN (US)) 27 février 2003 (2003-02-27) * page 12, dernier alinéa; revendications 1,35,46,90 * | 1-30 | A61K7/09 A61K7/13 A61K7/135 |
| A | EP 0 509 382 A (GRACE W R & CO) 21 octobre 1992 (1992-10-21) * revendications 1,6,10; tableau I * | 1,15-30 | |
| A | US 5 621 008 A (PTCHELINTSEV DMITRI) 15 avril 1997 (1997-04-15) * revendication 1 * | 15 | |
| A | US 2002/127194 A1 (DEVIN-BAUDOIN PRISCILLE ET AL) 12 septembre 2002 (2002-09-12) * exemple 1 * | 1,15-30 | |
| A | DATABASE CA [Online] CHEMICAL ABSTRACTS SERVICE, COLUMBUS, OHIO, US; KANEKO, YOHEI ET AL: "Biodegradable cleaning compositions with good disinfecting properties for skin and hair" XP002262925 extrait de STN Database accession no. 130:316420 * abrégé * & JP 11 092794 A (KAO CORP., JAPAN) 6 avril 1999 (1999-04-06) | 1,15-30 | **DOMAINES TECHNIQUES RECHERCHES (Int.Cl.7)** A61K |
| A | US 2003/032573 A1 (KINDERDINE SHERRIE L ET AL) 13 février 2003 (2003-02-13) * revendications 1,2,13 * | 1,15-30 | |
| A | US 5 786 313 A (SCHNEIDER JUERGEN ET AL) 28 juillet 1998 (1998-07-28) * revendication 1; tableau 1 * | 1,15-30 | |

Le présent rapport a été établi pour toutes les revendications

| Lieu de la recherche | Date d'achèvement de la recherche | Examinateur |
|---|---|---|
| La Haye | 13 septembre 2004 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 04 10 1243

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-09-2004

| Document brevet cité au rapport de recherche | | Date de publication | Membre(s) de la famille de brevet(s) | | | Date de publication |
|---|---|---|---|---|---|---|
| WO 03015732 | A | 27-02-2003 | US | 2003037384 | A1 | 27-02-2003 |
| | | | WO | 03015732 | A1 | 27-02-2003 |
| EP 0509382 | A | 21-10-1992 | US | 5362412 | A | 08-11-1994 |
| | | | AT | 173007 | T | 15-11-1998 |
| | | | CA | 2063118 | A1 | 18-10-1992 |
| | | | DE | 69227479 | D1 | 10-12-1998 |
| | | | DE | 69227479 | T2 | 10-06-1999 |
| | | | EP | 0509382 | A2 | 21-10-1992 |
| | | | ES | 2122974 | T3 | 01-01-1999 |
| US 5621008 | A | 15-04-1997 | US | 5728733 | A | 17-03-1998 |
| US 2002127194 | A1 | 12-09-2002 | FR | 2814066 | A1 | 22-03-2002 |
| | | | AU | 5992401 | A | 21-03-2002 |
| | | | BR | 0104082 | A | 07-05-2002 |
| | | | CA | 2356550 | A1 | 18-03-2002 |
| | | | CN | 1344538 | A | 17-04-2002 |
| | | | CZ | 20013325 | A3 | 17-07-2002 |
| | | | EP | 1188432 | A1 | 20-03-2002 |
| | | | HU | 0103718 | A2 | 28-11-2002 |
| | | | JP | 2002114651 | A | 16-04-2002 |
| | | | PL | 349734 | A1 | 25-03-2002 |
| | | | RU | 2216310 | C2 | 20-11-2003 |
| | | | ZA | 200106926 | A | 13-03-2002 |
| JP 11092794 | A | 06-04-1999 | AUCUN | | | |
| US 2003032573 | A1 | 13-02-2003 | AUCUN | | | |
| US 5786313 | A | 28-07-1998 | DE | 4319935 | A1 | 22-12-1994 |
| | | | AT | 196921 | T | 15-10-2000 |
| | | | AT | 257466 | T | 15-01-2004 |
| | | | AT | 226241 | T | 15-11-2002 |
| | | | CA | 2162122 | A1 | 22-12-1994 |
| | | | DE | 59409549 | D1 | 16-11-2000 |
| | | | DE | 59410198 | D1 | 21-11-2002 |
| | | | DE | 59410351 | D1 | 12-02-2004 |
| | | | DK | 703971 | T3 | 13-11-2000 |
| | | | DK | 781762 | T3 | 08-03-2004 |
| | | | DK | 846753 | T3 | 04-11-2002 |
| | | | WO | 9429421 | A1 | 22-12-1994 |
| | | | EP | 1334961 | A1 | 13-08-2003 |
| | | | EP | 0703971 | A1 | 03-04-1996 |
| | | | EP | 0781762 | A1 | 02-07-1997 |
| | | | EP | 0846753 | A1 | 10-06-1998 |

EPO FORM P0460

*Pour tout renseignement concernant cette annexe : voir Journal Officiel de l'Office européen des brevets, No.12/82*

**ANNEXE AU RAPPORT DE RECHERCHE EUROPEENNE RELATIF A LA DEMANDE DE BREVET EUROPEEN NO.** EP 04 10 1243

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-09-2004

| Document brevet cité au rapport de recherche | Date de publication | Membre(s) de la famille de brevet(s) | Date de publication |
|---|---|---|---|
| US 5786313 A | | EP 0976818 A1 | 02-02-2000 |
| | | ES 2151551 T3 | 01-01-2001 |
| | | ES 2184955 T3 | 16-04-2003 |
| | | JP 8511255 T | 26-11-1996 |
| | | US 6005141 A | 21-12-1999 |
| | | US 6008176 A | 28-12-1999 |

EPO FORM P0460

Pour tout renseignement concernant cette annexe : voir Journal Officiel de l'Office européen des brevets, No.12/82